# EUROPEAN PATENT APPLICATION

(11) **EP 4 683 485 A1**
(43) Date of publication of application: **21.01.2026**
(21) Application number: 23934622.4
(22) Date of filing: 28.04.2023
(51) Int. Cl.: H10K 85/50, H10K 30/40, H10K 30/88

(54) **PEROVSKITE PRECURSOR SOLUTION, PEROVSKITE THIN FILM, PEROVSKITE CELL AND ELECTRICAL APPARATUS**

(71) Applicant: Contemporary Amperex Technology Co., Limited, Ningde, Fujian 352100 (CN)
(72) Inventor: LI, Hanfang, Ningde, Fujian 352100 (CN); SU, Shuojian, Ningde, Fujian 352100 (CN); LIN, Xiangling, Ningde, Fujian 352100 (CN); ZHANG, Fan, Ningde, Fujian 352100 (CN); LIU, Zezheng, Ningde, Fujian 352100 (CN); GUO, Yongsheng, Ningde, Fujian 352100 (CN); CHEN, Guodong, Ningde, Fujian 352100 (CN)
(74) Representative: Ziebig Hengelhaupt Intellectual Property Attorneys Patentanwaltskanzlei PartGmbB
(86) International application number: PCT/CN2023/091707
(87) International publication number: WO 2024/221430

(57) **Abstract**

The present application provides a perovskite precursor solution, a perovskite thin film, a perovskite cell and an electrical apparatus. The perovskite precursor solution includes a surfactant, a structure of the surfactant includes a hydrophilic group, a hydrophobic group and a first functional group which are different from each other, the first functional group is a Lewis base group containing a lone pair, and the lone pair is present in at least one of an N atom and an S atom.

## Description

### TECHNICAL FIELD

The present application relates to the technical field of solar cells, in particular to a perovskite precursor solution, a perovskite thin film, a perovskite cell and an electrical apparatus.

### BACKGROUND

The statement here merely provides background information related to the present application, and does not necessarily constitute the prior art.

Perovskite solar cells are apparatuses that use a photoelectric conversion mechanism of a perovskite crystal material to convert solar energy into electrical energy, are current third-generation solar cells, have many advantages, such as high photoelectric conversion efficiency, simple manufacturing process, and low production cost, and have been extensively studied in recent years. Despite this, there is still some distance to go before perovskite cells can be commercialized on a large scale, especially since the energy conversion efficiency of perovskite thin films is one of the important factors restricting the industrialization of the perovskite cells.

### SUMMARY OF THE INVENTION

In view of the above problem, the present application provides a perovskite precursor solution, a perovskite thin film, a perovskite cell and an electrical apparatus. This perovskite precursor solution may significantly improve energy conversion efficiency of the cell.

In a first aspect, the present application provides a perovskite precursor solution, including a perovskite precursor material, a solvent and a surfactant, a structure of the surfactant including a hydrophilic group, a hydrophobic group and a first functional group, wherein the first functional group is a Lewis base group containing a lone pair, and the lone pair is present in at least one of an N atom and an S atom; and
the first functional group, the hydrophilic group and the hydrophobic group are different from each other.

In the structure of the surfactant having the hydrophilic group and the hydrophobic group, the lone pair may be provided by a specific type of atom (including at least one specific atom of N and S), so that the surfactant has suitable Lewis base property. On the one hand, wettability of the perovskite precursor solution on asubstrate surface can be improved, and on the other hand, the surfactant can more stably complex with a divalent metal ion in the perovskite precursor material and better passivate multiple defects, such as lead vacancy and iodine vacancy in perovskite. By introducing this surfactant of the special structure into the perovskite precursor solution, the uniformity of the perovskite thin film can be improved, and the energy conversion efficiency of the cell can be improved. In addition, the perovskite precursor solution is conducive to preparation of large-area (e.g, ≥1 cm²) perovskite thin films.

In some embodiments, the first functional group includes one or more of -NR¹¹-NR¹²R¹³ and - SH; wherein R¹¹ and R¹² are each independently H or hydrocarbyl; R¹³ is H, hydrocarbyl, or hydrocarbyl substituted with a monovalent hydrophilic group;
optionally, R¹¹ and R¹² are each independently H or alkyl, further optionally, R¹¹ and R¹² are each independently H or C₁₋₆ alkyl, still further optionally, R¹¹ and R¹² are each independently H or C₁₋₃ alkyl; still further optionally, R¹¹ and R¹² are each independently H or methyl; and still further optionally, R¹¹ and R¹² are both H;
optionally, R¹³ is H, alkyl or alkyl substituted with a monovalent hydrophilic group, further optionally, the alkyl or the alkyl substituted with the monovalent hydrophilic group is each independently C₁₋₂₀ alkyl, still further optionally C₁₋₁₈ alkyl, still further optionally C₁₋₁₃ alkyl, still further optionally C₁₋₁₂ alkyl, still further optionally C₁₋₁₀ alkyl, still further optionally C₁₋₈ alkyl, still further optionally C₁₋₆ alkyl, still further optionally C₁₋₄ alkyl, still further optionally C₁₋₃ alkyl, still further optionally methyl;
optionally, R¹³ is H or hydrocarbyl, further optionally H or alkyl, still further optionally H or C₁₋₂₀ alkyl, still further optionally H or C₁₋₁₈ alkyl, still further optionally H or C₁₋₁₃ alkyl, still further optionally H or C₁₋₁₂ alkyl, still further optionally H or C₁₋₁₀ alkyl, still further optionally H or C₁₋₈ alkyl, still further optionally H or C₁₋₆ alkyl, still further optionally H or C₁₋₄ alkyl, still further optionally H or C₁₋₃ alkyl, still further optionally H or methyl;
optionally, examples of the monovalent hydrophilic group may include, but are not limited to, one or more of: carboxyl, a sulfonic acid group, a sulfonate salt, a sulfuric acid group, a sulfate salt, a phosphoric acid group, a phosphate salt, a monohydrogen phosphate group, a monohydrogen phosphate salt, a primary amino group, a primary amino salt, a secondary amino group, a secondary amino salt, a quaternary ammonium salt, -CONH₂, and hydroxyl; and
optionally, R¹¹, R¹² and R¹³ are all H; still further optionally, the first functional group is selected from one or both of -NH-NH₂ and -SH.

A group having at least one of an N-N structure and -SH structure can be used as the first functional group, at this moment, more suitable Lewis base property can be provided, thereby playing a better role in improving the wettability of the perovskite precursor solution and reducing the vacancy defects in the perovskite, thereby better improving the uniformity of the perovskite thin film, which is also more beneficial to improving the energy conversion efficiency of the cell.

In some embodiments, the hydrophilic group includes one or more of carboxyl, a sulfonic acid group, a sulfonate salt, a sulfuric acid group, a sulfate salt, a phosphoric acid group, a phosphate salt, a monohydrogen phosphate group, a monohydrogen phosphate salt, a primary amino group, a primary amino salt, a secondary amino group, a secondary amino salt, a divalent tertiary amino group, a quaternary ammonium salt, -CONH₂, hydroxyl, an ether group, an ester group, -CONH- and a divalent phosphate ester group;
optionally, the sulfonate salt, the sulfate salt, the phosphate salt and the monohydrogen phosphate salt are alkali metal salts of corresponding acids;
optionally, the primary amino salt and the secondary amino salt are respectively salts formed by corresponding amine and acid; optionally, the acid is organic acid or inorganic acid; the organic acid includes one or more of carboxylic acid, phosphonic acid, and sulfonic acid; the inorganic acid includes one or more of hydrohalic acid, phosphoric acid, and sulfuric acid; and optionally, the inorganic acid includes one or more of hydrochloric acid, hydroiodic acid, hydrobromic acid, hydrofluoric acid, and sulfuric acid; and
optionally, the hydrophilic group includes at least one of quaternary ammonium ions and alkali metal ions.

In some embodiments, the hydrophilic group includes one or more of *-COOH, *-S(=O)₂OH, *-S(=O)₂OM, *-O-S(=O)₂OH, *-O-S(=O)₂OM, *-O-(O=)P(OH)₂, 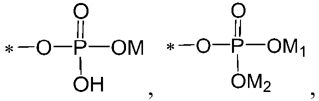 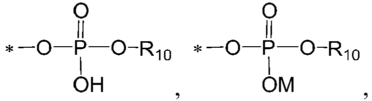 *-NH₂, *-NH₂·n₂A_{cd}, *-NHR₀, *-NHR₀·n₁A_{cd}, 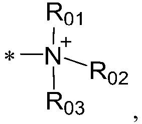 *-CONH₂, *-OH and a hydrophilic linker L₀; wherein the hydrophilic linker L₀ includes one or more of *-CONH-*, *-NHCO-*, *-C(=O)O-*, *-O-C(=O)-*, *-O-(O=)P(OM₀₁)-O-*, *-NH-* 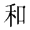 *-O-*;
wherein any "*" indicates a connection site to a carbon atom;
any M is independently an alkali metal ion;
any M₁ and any M₂ are each independently an alkali metal ion;
any R₁₀ is independently hydrocarbyl;
any A_{cd} is independently an acid molecule; n₂ is 1 or 2; n₁ is 1;
any R₀ is independently hydrocarbyl or substituted hydrocarbyl; the substituted hydrocarbyl is substituted by one or more hydrophilic groups;
R₀₁ is alkyl; R₀₂ is hydrocarbyl; R₀₃ is alkyl; and
M₀₁ is absent or is H or an alkali metal ion.

In some embodiments, the surfactant satisfies any one or more of the following features:
any M is independently lithium, sodium or potassium;
any M₁ and any M₂ are each independently lithium, sodium or potassium;
any R₁₀ is independently C₁₋₁₀ alkyl, optionally C₁₋₈ alkyl, further optionally C₁₋₆ alkyl, still further optionally C₁₋₄ alkyl, still further optionally C₁₋₃ alkyl;
any A_{cd} is independently an organic acid or inorganic acid molecule; the organic acid includes one or more of carboxylic acid, phosphonic acid, and sulfonic acid; the inorganic acid includes one or more of hydrohalic acid, phosphoric acid, and sulfuric acid; and optionally, the inorganic acid includes one or more of hydrochloric acid, hydroiodic acid, hydrobromic acid, hydrofluoric acid, and sulfuric acid; and
any R₀ is independently alkyl; optionally, any R₀ is independently C₁₋₃ alkyl; further optionally, R₀ is methyl;
R₀₁ is C₁₋₃ alkyl; optionally, R₀₁; is methyl;
R₀₂ is a C₁₋₈ alkyl or C₁₋₃ alkylene substituted with a benzene ring, and the benzene ring is phenyl or phenyl substituted with 1-4 C₁₋₃ alkyl; optionally, R₀₂ is methyl or benzyl;
R₀₃ is C₁₋₃ alkyl; optionally, R₀₃ is methyl;
M₀₁ is absent or is H, lithium, sodium or potassium; and
the monovalent hydrophilic group is connected to at least one side of the hydrophilic linker L₀.

The surfactant provided in the present application may contain various types of hydrophilic groups, so that surface tension of the surfactant can be more flexibly adjusted, and the wettability of the perovskite precursor solution and the passivation effect on the vacancy defects in the perovskite can be more flexibly exerted. When the structure of the surfactant includes an alkali metal salt hydrophilic group, it is beneficial to further passivate interface defects of a perovskite layer. When the structure of the surfactant includes at least one of a primary amino group, a secondary amino group or tertiary aminoin, it is beneficial to passivate bulk phase defects. When the structure of the surfactant includes a quaternary ammonium salt, it is beneficial to control a nucleation rate and promote grain growth. When acid molecules are complexed in the structure of the surfactant, it is beneficial to the uniformity of large-area film formation of the perovskite layer. The hydrophilic group can be not only a monovalent hydrophilic group located at an end group, but also a multivalent group as a linker, and can also be a combination of monovalent or multivalent hydrophilic groups, so that the hydrophilic group can be coordinated with the hydrophobic group and the first functional group by adjusting a position, number and other parameters of the hydrophilic group, thereby giving the perovskite precursor solution good wettability, reducing the vacancy defects in the perovskite, improving the uniformity of the perovskite thin film, and further improving the energy conversion efficiency of the cell.

In some embodiments, the hydrophobic group includes a C₈₋₂₀ carbon chain;
optionally, the C₈₋₂₀ carbon chain is a linear or branched structure;
optionally, a main chain atom length of the C₈₋₂₀ carbon chain is in a range from 6 to 20; further optionally, the main chain atom length of the C₈₋₂₀ carbon chain is in a range from 8 to 20; still further optionally, the main chain atom length of the C₈₋₂₀ carbon chain is in a range from 8 to 18; still further optionally, the C₈₋₂₀ carbon chain is a C₁₀₋₂₀ carbon chain with a main chain atom length ranging from 10 to 18;
optionally, the C₈₋₂₀ carbon chain is a saturated structure or an unsaturated structure;
optionally, the C₈₋₂₀ carbon chain is an aromatic chain or an aliphatic chain; and
optionally, a molecular structure of the surfactant includes one or more C₈₋₂₀ carbon chains.

By introducing the hydrophobic group of a certain size into the structure of the surfactant, the hydrophobic group can cooperate with the hydrophilic group and the first functional group to form a hydrophilic-hydrophobic balance that is more suitable for the perovskite precursor solution of the present application. While playing the role of the lone pair in the first functional group, a perovskite precursor solution system can be made more stable and better dispersed, with better wettability to the substrate, thereby better improving the uniformity of the perovskite thin film.

In some embodiments, the hydrophobic group further includes one or more of arylene Ar₀ and arylalkyl ArA;
optionally, the arylene Ar₀ is C₆₋₁₈ arylene, and further optionally, the arylene Ar₀ is phenylene or phenylene substituted with one or more C₁₋₃ alkyl; and
optionally, the aralkyl ArA is C₁₋₁₈ alkyl substituted with one or more aryl Ar₁, wherein any one of the aryl Ar₁ is independently phenyl or phenyl substituted with one or more C₁₋₄ alkyl; further optionally, any one of the aryl Ar₁ is independently phenyl or phenyl substituted with one or more C₁₋₃ alkyl; still further optionally, any one of the aryl Ar₁ is independently phenyl or benzyl; and further optionally, the aralkyl ArA is benzyl.

An aromatic ring may be introduced into the hydrophobic structure of the surfactant, which is more conducive to the interaction between surfactant molecules and perovskite components, and is conducive to better dissolution in the perovskite precursor solution.

In some embodiments, in one molecule of the surfactant, the number of the hydrophilic groups, the hydrophobic groups and the first functional groups is each independently one or more;
optionally, in one molecule of the surfactant, the number of the first functional groups is 1 or 2-5; and
optionally, in one molecule of the surfactant, the number of the hydrophilic groups is 1 or 2-5.

The number of the hydrophilic groups, the hydrophobic groups and the first functional groups in the surfactant can be adjusted respectively, so that the surfactant can simultaneously obtain a suitable hydrophilic-hydrophobic balance and Lewis basicity of the lone pair in a wider range.

In some embodiments, a molar percentage of the first functional group relative to a divalent metal ion in the perovskite precursor material is in a range from 0.001 mol% to 5 mol%; and
optionally, the molar percentage of the first functional group relative to the divalent metal ion in the perovskite precursor material is in a range from 0.1 mol% to 2.5 mol%.

The amount of the first functional group can be adjusted according to the usage amount of the divalent metal ion in the perovskite precursor material, so as to better realize the synergistic effect of the surfactant and the perovskite precursor material while both improving the wettability of the perovskite precursor solution and reducing the vacancy defects in the perovskite, thereby better improving the uniformity of the perovskite thin film, which is also more beneficial to improving the energy conversion efficiency of the cell.

In some embodiments, the first functional group and the hydrophilic group are each independently connected to a carbon atom of the hydrophobic group.

In some embodiments, the surfactant includes one or more of the following compounds: 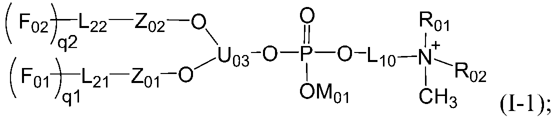 in formula (I-1), q1 and q2 are each independently 0 or a positive integer, q1+q2≥1, and L₂₁ and L₂₂ are each independently polyvalent hydrocarbyl having a main chain atom length of 8 to 20; Z₀₁; and Z₀₂ are each independently a covalent bond, carbonyl or -NHC(=O)-*, wherein * points to U₀₃; U₀₃ is trivalent hydrocarbyl; M₀₁ is absent or is H or an alkali metal ion; L₁₀ is C₁₋₆ alkylene; R₀₁; is alkyl; R₀₂ is hydrocarbyl; in one molecule, at least one of F₀₁ and F₀₂ is the first functional group, and the other is independently H or the first functional group; 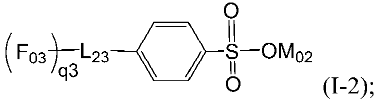 in formula (I-2), M₀₂ is an alkali metal ion; 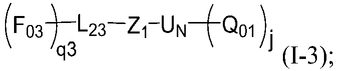 in formula (I-3), Z₁ is a covalent bond or a linker Z₁₀, wherein Z₁₀ is selected from any one of the following groups: -CO-NH, -NH-CO-, -C(=O)-O-, -O-C(=O)-, -NH-C(=O)-O-, -O-C(=O)-NH- and -O-; U_{N} is a j+1 valent group; j is a positive integer; any Q₀₁ is independently -OH or -COOM₀₃, M₀₃ is H or an alkali metal ion; 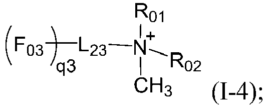 in formula (I-4), R₀₁ is alkyl; R₀₂ is hydrocarbyl;
in formula (I-2), formula (I-3), formula (I-4) and formula (I-5), F₀₃ is respectively and independently the first functional group; and
in formula (I-2), formula (I-3), formula (I-4) and formula (I-5), q3 is each independently an integer greater than 1, and L₂₃ is each independently polyvalent hydrocarbyl having a main chain atom length of 8 to 20.

In some embodiments, the surfactant satisfies any one or more of the following features:
in formula (I-1), L₂₁ and L₂₂ are each independently a linear or branched type; optionally, L₂₁ and L₂₂ are each independently linear C₈₋₂₀ alkylene or branched C₈₋₂₀polyvalent hydrocarbyl; further optionally, L₂₁ and L₂₂ are each independently linear C₈₋₂₀ hydrocarbylene; still further optionally, L₂₁ and L₂₂ are each independently linear C₈₋₂₀ alkylene or linear C₈₋₂₀ alkenylene; still further optionally, L₂₁ and L₂₂ are each independently linear C₁₀₋₁₈ alkylene or linear C₁₀₋₁₈alkenylene; still further optionally, L₂₁ and L₂₂ are each independently linear C₁₂₋₁₈ alkylene or linear C₁₂₋₁₈ alkenylene;
in formula (I-1), U₀₃ is trivalent alkyl or 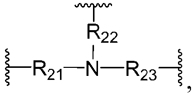 and R_{21,} R₂₂ and R₂₃ are each independently alkylene; optionally, U₀₃ is trivalent C₂₋₁₀ alkyl or trivalent C₃₋₁₀ tertiary amino, further optionally, U₀₃ is -CH₂₋CR₀₄(-)-CH₂-, >CH-L₀₄- or N(-CH₂CH₂-)₃, R₀₄ is H or C₁₋₄ alkyl (R₀₄ is further optionally H, methyl or ethyl, still further optionally H), L₀₄ is C₁₋₆ alkylene (L₀₄ is further optionally C₁₋₄ alkylene, still further optionally C₁₋₄ alkylene, still further optionally methylene, 1,2-ethylidene, 1,3-propylidene or 1,4-butylidene); optionally, R_{21,} R₂₂ and R₂₃ are each independently C₁₋₄ alkylene, further optionally, R₂₁ R₂₂ and R₂₃ are each independently C₁₋₃ alkylene, still further optionally, R₂₁, R₂₂ and R₂₃ are each independently methylene or ethylidene, and still further optionally, R₂₁, R₂₂ and R₂₃ are all ethylidene;
in formula (I-1), M₀₁ is absent or is H, a lithium ion, a sodium ion or a potassium ion;
in formula (I-1), L₁₀ is C₁₋₄ alkylene, optionally ethylidene or propylidene, afurther optionally ethylidene;
in formula (I-1), R₀₁; is C₁₋₃ alkyl; optionally, R₀₁; is methyl;
in formula (I-1), R₀₁; is C₁₋₈ alkyl or C₁₋₃ alkylene substituted with a benzene ring, wherein the benzene ring is phenyl or phenyl substituted with 1-4 C₁₋₃ alkyl; optionally, R₀₂ is a methyl or benzyl;
in formula (I-2), M₀₂ is H, a lithium ion, a sodium ion or a potassium ion;
n formula (I-3), U_{N} is polyvalent hydrocarbyl containing 0, 1 or more hydrophilic linkers L₀₁, optionally, U_{N} is polyvalent saturated hydrocarbyl containing 0, 1 or more hydrophilic linkers L₀₁, any of the hydrophilic linkers L₀₁ is independently *-CONH-*, *-NHCO-*, *-C(=O)O-*, *-O-C(=O)-*, *-O-(O=)P(OM₀₁)-O-* or *-O-*, wherein * represents a connection site to a carbon atom, and M₀₁ is absent or is H, a lithium ion, a sodium ion or a potassium ion; alternatively, U_{N} is trivalent alkyl or tetravalent alkyl, optionally, the number of carbon atoms of U_{N} is in a range from 3 to 10, further optionally in a range from 3 to 6, further optionally 3, 4 or 5, still further optionally, U_{N} is -CH₂₋CR₀₅(-)-CH₂-, R₀₅ is H, methyl or ethyl, and still further optionally, R₀₅ is H; alternatively, U_{N} is trivalent tertiary amino, optionally trivalent C₃₋₁₀ tertiary amino, and further optionally N(-CH₂CH₂-)₃;
in formula (I-3), the number of non-hydrogen atoms of U_{N} is in a range from 2 to 40, optionally in a range from 2 to 30, further optionally in a range from 2 to 25, still further optionally in a range from 2 to 20, still further optionally in a range from 2 to 18, still further optionally in a range from 2 to 15, still further optionally in a range from 2 to 12, still further optionally in a range from 2 to 10, still further optionally in a range from 2 to 8, still further optionally in a range from 2 to 6, still further optionally 2, 3, 4 or 5;
in formula (I-3), j is an integer selected from 1 to 5, optionally an integer selected from 1 to 4, further optionally 1, 2 or 3;
in formula (I-4), R₀₁; is C₁₋₃ alkyl; optionally, R₀₁; is methyl;
in formula (I-4), R₀₁; is C₁₋₈ alkyl or C₁₋₃ alkylene substituted with a benzene ring, wherein the benzene ring is phenyl or phenyl substituted with 1-4 C₁₋₃ alkyl; optionally, R₀₂ is a methyl or benzyl;
in formula (I-2), formula (I-3) and formula (I-4), L₂₃ is respectively and independently a linear or branched type; optionally, L₂₃ is respectively and independently linear C₈₋₂₀ hydrocarbylene or branched C₈₋₂₀ polyvalent hydrocarbyl; further optionally, L₂₃ is respectively and independently linear C₈₋₂₀ alkylene or linear C₈₋₂₀ alkenylene; still further optionally, L₂₃ is respectively and independently linear C₁₀₋₁₈ alkylene or linear C₁₀₋₁₈ alkenylene; still further optionally, L₂₃ is respectively and independently linear C₁₂₋₁₈ alkylene or linear C₁₂₋₁₈ alkenylene.

In some embodiments, the surfactant includes one or more of the following compounds: 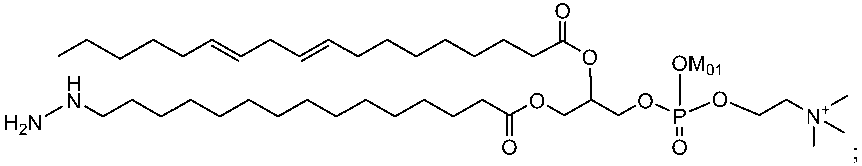 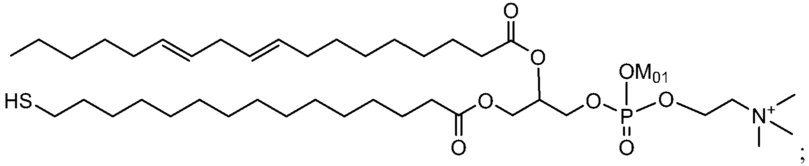 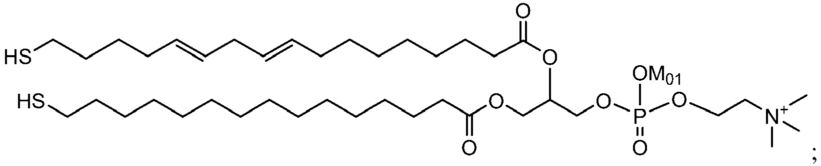 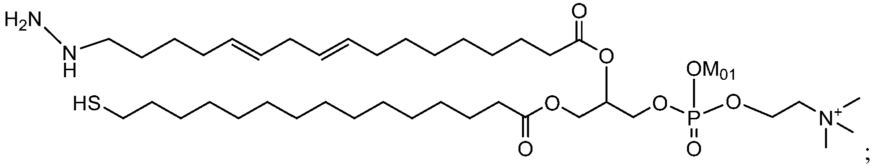 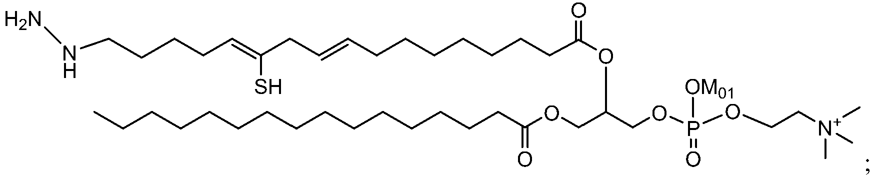 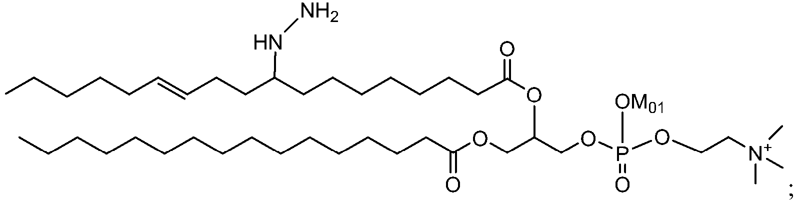 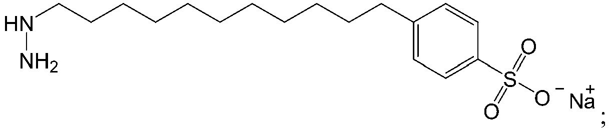 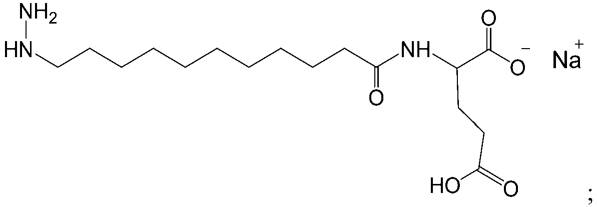 wherein M₀₁ is absent or is H or an alkali metal ion.

For the surfactant containing a quaternary ammonium salt group, while adjusting film formation, the surfactant can interact with a perovskite intermediate phase, so as to adjust a crystallization nucleation rate and crystallization speed, and promote the grain growth.

For the surfactant containing a monohydrogen phosphate group, an interface between the perovskite layer and a hole transport layer can be passivated.

For the branched surfactant containing at least two hydrophobic carbon chains, the damage of water to the perovskite layer can be avoided.

For the linear surfactant containing sulfonate, bulk phase defects are reduced and short-circuit current density (Jsc) is increased.

For the surfactant whose hydrophilic group includes one or more hydroxyl, the solubility of the surfactant in the precursor solution can be increased, thereby improving the uniformity of large-area coating.

For the surfactant whose hydrophilic group includes one or more carboxyl, the bulk phase defects can be reduced, and device stability can be improved.

In some embodiments, the molar percentage of the surfactant relative to the divalent metal ion in the perovskite precursor material is in a range from 0.001 mol% to 5mol%;
optionally, the molar percentage of the surfactant relative to the divalent metal ion in the perovskite precursor material is in a range from 0.001% to 2.5%;
further optionally, the molar percentage of the surfactant relative to the divalent metal ion in the perovskite precursor material is in a range from 0.01% to 1%; and
still further optionally, the molar percentage of the surfactant relative to the divalent metal ion in the perovskite precursor material is in a range from 0.01% to 0.5%.

The amount of the first functional group can be adjusted by adjusting the content of the surfactant, so as to better realize the synergistic effect of the surfactant and the perovskite precursor material while taking into account improving the wettability of the perovskite precursor solution and reducing the vacancy defects in the perovskite, thereby better improving the uniformity of the perovskite film, which is also more beneficial to improving the energy conversion efficiency of the battery.

In some embodiments, the solvent includes a first solvent and a second solvent, and a boiling point of the first solvent is lower than a boiling point of the second solvent.

In some embodiments, the first solvent includes one or more of N,N-dimethylformamide, 2-methoxyethanol and acetonitrile;
the second solvent includes one or more of N-methylpyrrolidone, diphenyl sulfoxide, and dimethylpropyleneurea.

In some embodiments, a volume ratio of the first solvent to the second solvent is in a range from 3 to 10;
optionally, the volume ratio of the first solvent to the second solvent is in a range from 3 to 5.

Solvents with different boiling points can be used in combination to adjust a solvent evaporation rate and thus adjust and control the morphology of the perovskite thin film when preparing the perovskite thin film, thereby better improving the uniformity of the thin film and being more conducive to improving the energy conversion efficiency of the perovskite cell.

In a second aspect, the present application provides a perovskite thin film, prepared by coating and annealing the perovskite precursor solution described in the first aspect of the present application or at least containing a non-solvent component in the perovskite precursor solution described in the first aspect of the present application.

The perovskite thin film prepared using the perovskite precursor solution described in the first aspect of the present application has good contact with a substrate, the thin film is uniform and has few defects, and the corresponding perovskite cell has high energy conversion efficiency.

In some embodiments, an area of the perovskite thin film is greater than or equal to 1cm².

It is difficult to obtain a relatively uniform perovskite thin film for a large-area perovskite thin film (e.g ≥1 cm²). Compared with a perovskite precursor solution using a traditional surfactant, the perovskite precursor solution containing the surfactant having the aforementioned first functional group provided in the present application has significant advantages in improving the uniformity of the large-area perovskite thin film.

In a third aspect, the present application provides a perovskite cell including the perovskite thin film described in the second aspect of the present application.

In some embodiments, an area of the perovskite thin film is greater than or equal to 1cm²;
optionally, the area of the perovskite thin film is greater than or equal to 4 cm².

In a fourth aspect, the present application provides an electrical apparatus, including the perovskite cell described in the third aspect of the present application.

Details of one or more examples of the present application are provided in the accompanying drawings and descriptions below. Other features, objectives, and advantages of the present application will become apparent from the specification, drawings, and claims.

### DESCRIPTION OF DRAWINGS

In order to better describe and illustrate the embodiments or examples of the applications disclosed herein, reference may be made to one or more of the figures. The additional details or examples used to describe the drawings should not be considered as limiting the scope of the disclosed applications, currently described embodiments or examples, and any one of currently understood preferred embodiments of these applications. Moreover, in all of the figures, identical parts are represented by identical reference numerals. In the figures:
FIG. 1 is a schematic diagram of a perovskite cell according to an embodiment of the present application, wherein the cell includes a first electrode, a first transport layer, a perovskite layer, a second transport layer, and a second electrode;
FIG. 2 is a schematic diagram of a perovskite cell according to an embodiment of the present application, wherein the cell includes a base layer, a first electrode, a first transport layer, a perovskite layer, a second transport layer, and a second electrode;
FIG. 3 is a schematic diagram of a perovskite cell according to an embodiment of the present application; and
FIG. 4 is a schematic diagram of an electrical apparatus in which a perovskite cell serves as a power generation device according to an embodiment of the present application.

Explanation of the reference numerals : 100 is a perovskite cell; 110 is a substrate layer; 120 is a first electrode; 130 is a first transport layer; 140 is a perovskite layer; 150 is a second transport layer; 160 is a second electrode; P1 is a first nick region; P2 is a second nick region; P3 is a third nick region; and 20 is an electrical apparatus.

### DETAILED DESCRIPTION

Some embodiments of a perovskite precursor solution, a perovskite thin film, a perovskite cell and an electrical apparatus of the present application are disclosed below with reference to the detailed description of drawings as appropriate. However, there may be cases where unnecessary detailed descriptions are omitted. For example, there are cases where detailed descriptions of well-known items and repeated descriptions of actually identical structures are omitted. This is to avoid unnecessary redundancy in the following descriptions and to facilitate understanding by those skilled in the art. In addition, the drawings and subsequent descriptions are provided for those skilled in the art to fully understand the present application, and are not intended to limit the subject matter recited in the claims.

The "range" disclosed in the present application is defined in a form of a lower limit and an upper limit. A given range is defined by selecting a lower limit and an upper limit, and the selected lower limit and upper limit define the boundaries of the particular range. A range defined in this manner may be inclusive or exclusive of end values, and may be arbitrarily combined, that is, any lower limit may be combined with any upper limit to form a range. For example, if the ranges 60-120 and 80-110 are listed for specific parameters, it is understood that the ranges 60-110 and 80-120 are also expected. Additionally, if the minimum range values 1 and 2 are listed, and if the maximum range values 3, 4 and 5 are listed, the following ranges are all contemplated: 1-3, 1-4, 1-5, 2- 3, 2-4 and 2-5. In the present application, unless stated otherwise, the numerical range "a-b" represents an abbreviated representation of any combination of real numbers between a to b, where both a and b are real numbers. For example, the numerical range "0-5" means that all the real numbers between "0-5" have been enumerated herein, and "0-5" is just an abbreviated representation of combinations of these numerical values. In addition, when a parameter is expressed as an integer greater than or equal to 2, it is equivalent to enumerating that the parameter is, for example, an integer of 2, 3, 4, 5, 6, 7, 8, 9, 10, 11, 12, or the like. For example, when a parameter is expressed as an integer selected from "2-10", it is equivalent to listing an integer of 2, 3, 4, 5, 6, 7, 8, 9, and 10.

Unless otherwise specified, all embodiments and optional embodiments of the present application may be combined with each other to form new technical solutions.

Unless otherwise particularly stated, all steps in the present application may be performed sequentially or may be performed randomly, and are preferably performed sequentially. For example, the method comprises steps (a) and (b), meaning that the method may comprise steps (a) and (b) performed sequentially, or may comprise steps (b) and (a) performed sequentially. For example, the reference to the method may further comprise step (c), which means that step (c) may be added to the method in any order, for example, the method may comprise steps (a), (b) and (c), or may comprise steps (a), (c) and (b), or may comprise steps (c), (a) and (b), and so on.

Unless otherwise particularly stated, the terms "including," "containing," and "comprising" mentioned in the present application may be open-ended, or may be closed-ended. For example, the "including," "containing," and "comprising" may mean that other unlisted members, elements or method steps may be further included or comprised, or only the listed members, elements or method steps may be included or comprised.

Unless otherwise particularly stated, the term "or" is inclusive in the present application. For example, the phrase "A or B" means "A, B, or both A and B." Further, the condition "A or B" is satisfied under any one of the following conditions: A is true (or present) and B is false (or absent); A is false (or absent) and B is true (or present); or both A and B are true (or present).

In the present application, unless otherwise specified, A (such as B) means that B is a non-limiting example of A, and may be understood as that A is not limited to B.

The "plurality", "multiple" and the like involved in the present application, unless otherwise specified, refer to a number greater than 2 or equal to 2. For example, the "one or more" means one or greater than or equal to two.

The "combination thereof", "any combination thereof", "any combination mode thereof", etc. used in the present application include all suitable combination modes of any two or any more than two of the listed items.

In the present application, the "suitable" in the "suitable combination mode," "suitable mode," "any suitable mode," etc. shall be subject to a technical solution capable of implementing the present application.

In the present application, the "preferable", "better", "preferred", and "suitable" are only used to describe an implementation or embodiment with better effects, and should be understood as that they do not constitute a limitation to the scope of protection of the present application. If a plurality of "preferable" appear in a technical solution, unless otherwise specified and in the case of no contradiction or mutually restrictive relationship, each "preferable" is independent.

In the present application, the "optionally" and "optional" mean dispensable, that is, they mean any one in two parallel solutions of "presence" or "absence." If a plurality of "optional" appear in a technical solution, unless otherwise specified and in the case of no contradiction or mutually restrictive relationship, each "optional" is independent.

In the present application, the "further," "still further," "particularly", etc. are used for descriptive purposes and show differences of the different technical solutions in content, but should not be construed as limiting the scope of protection of the present application.

In the present application, the terms such as "first," "second," "third," and "fourth" in the phrases such as "first aspect," "second aspect," "third aspect," and "fourth aspect" are used only for descriptive purposes, and can neither be construed as indicating or implying a relative importance or number, nor be construed as implicitly indicating the importance or number of indicated technical features. Moreover, the "first," "second," "third," "fourth," etc. only serve the purpose of non-exhaustive enumeration and description, and should be understood as that they do not constitute a closed limitation to the quantity.

In the present application, the term "room temperature" generally refers to a temperature ranging from 4°C to 35°C, and may refer to 20°C±5°C. In some examples of the present application, the room temperature refers to a temperature ranging from 20°C to 30°C.

In the present application, if a unit involving a data range is only followed by the right endpoint, it means that the units of the left endpoint and the right endpoint are identical. For example, 3 to 5 h or 3-5 h means that the units of the left endpoint "3" and the right endpoint "5" are both h (hour).

The weight of the relevant components mentioned in the description of embodiments of the present application may not only refer to the content of each component, but also indicate the proportional relationship in weight between the components. Therefore, as long as the content of the relevant components in the description of embodiments of the present application is proportionally enlarged or reduced, it is within the scope disclosed in the description of embodiments of the present application. Further, the weight in the specification of the examples of the present application may be in µg, mg, g, kg, or other mass units known in the chemical industry.

In the present application, for an atom, a group or a compound residue participating in forming a covalent bond, its valence refers to the number of connection sites of the atom, the group or the compound residue participating in forming the covalent bond. For example, valence of alkyl (-CH₃) is monovalent, and valence of alkylene (-CH₂-) is divalent. For example, valence of groups such as -OH, -COOH, -CN, and -NHNH₂ is monovalent, while valence of -NHNH- is divalent. Those skilled in the art can understand the difference between this "valence" and a charge state of an ion.

In the present application, the "charge state of the ion" refers to the charge number of the ion, which can carry either a positive charge or a negative charge. For example, a positive charge state of a ferric ion (Fe³⁺) is 3, and a negative charge state of an iodine anion (I⁻) is 1.

In the present application, unless otherwise specified, "hydrocarbyl" refers to a monovalent or polyvalent group composed of a carbon atom and a hydrogen atom, and refers to a group formed when the corresponding hydrocarbon loses one or more hydrogen atoms, forming a corresponding covalent connection site at the position where the hydrogen atom is lost. If the valence of the "hydrocarbyl" is not directly or indirectly specified, it generally refers to monovalent hydrocarbyl. For example, references to "alkyl", "cycloalkyl", "aryl", "heteroalkyl", "heterocycloalkyl" and "heteroaryl", without direct or indirect indication of the valence, generally refer to monovalent alkyl, monovalent cycloalkyl, monovalent aryl, monovalent heteroalkyl, monovalent heterocycloalkyl and monovalent heteroaryl, respectively.

In the present application, "multivalent hydrocarbyl" refers to a multivalent group formed by the corresponding hydrocarbon losing the plurality of hydrogen atoms (such as ≥2, further such as 2, 3 and 4).

In the present application, Arabic numerals may be used as subscripts to indicate the number of atoms. For example, C₈₋₂₀ hydrocarbyl represents hydrocarbyl having 8 to 20 carbon atoms.

In the present application, unless otherwise specified, "hydroxyl" refers to - OH.

In the present application, the term "hydrocarbon" or "hydrocarbon compound" refers to a compound composed of carbon atoms and hydrogen atoms.

In the present application, unless otherwise specified, "hydrocarbyl" refers to a monovalent residue formed by the hydrocarbon compound losing one hydrogen atom.

In the present application, unless otherwise specified, the term "alkyl" refers to a monovalent residue formed by the loss of one hydrogen atom from saturated hydrocarbon containing a primary (normal) carbon atom, a secondary carbon atom, a tertiary carbon atom, a quaternary carbon atom, or a combination thereof. Phrases containing this term, for example, "C₁₋₉ alkyl" refers to alkyl containing 1 to 9 carbon atoms, and may independently be C₁ alkyl, C₂ alkyl, C₃ alkyl, C₄ alkyl, C₅ alkyl, C₆ alkyl, C₇ alkyl, C₈ alkyl, or C₉ alkyl each time it appears. Suitable examples include, but are not limited to, methyl (Me, -CH₃), ethyl (Et, -CH₂CH₃), 1-propyl (n-Pr, n-propyl, - CH₂CH₂CH₃), 2-propyl (i-Pr, i-propyl, -CH(CH₃)₂), 1-butyl (n-Bu, n-butyl, -CH₂CH₂CH₂CH₃), 2-methyl-1-propyl(i-Bu, i-butyl, -CH₂CH(CH₃)₂), 2-butyl (s-Bu, s-butyl, -CH(CH₃)CH₂CH₃), 2-methyl-2-propyl (t-Bu, t-butyl, -C(CH₃)₃), 1-pentyl (n-pentyl, -CH₂CH₂CH₂CH₂CH₃), 2-pentyl (-CH(CH3)CH2CH2CH3), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), 1-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃, and octyl (-(CH₂)₇CH₃).

In the present application, unless otherwise specified, "alkylene" refers to hydrocarbyl having two monovalent group centers derived from alkane by removing two hydrogen atoms (or derived from alkyl by losing another hydrogen atom), which may be saturated branched alkyl or saturated linear alkyl. For example, "C₁-C₉ alkylene" or "C₁₋₉ alkylene" refers to an alkyl moiety containing 1 to 9 carbon atoms, which may independently be C₁ alkylene, C₂ alkylene, C₃ alkylene, C₄ alkylene, C₅ alkylene, C₆ alkylene, C₇ alkylene, C₈ alkylene, or C₉ alkylene each time it appears. Suitable examples include, but are not limited to, methylene (-CH₂-), 1,1-ethyl (-CH(CH₃)-), 1,2-ethyl (-CH₂CH₂-), 1,1-propyl (-CH(CH₂CH₃)-), 1,2-propyl (-CH₂CH(CH₃)-), 1,3-propyl (-CH₂CH₂CH₂-), and 1,4-butyl (-CH₂CH₂CH₂CH₂-).

In the present application, unless otherwise specified, "aromatic-ring hydrocarbon compound" refers to a hydrocarbon compound containing an aromatic ring. The aromatic ring may or may not have a substituent. When the aromatic ring has the substituent, the number of the substituents may be one or more, and the substituents may be aromatic or non-aromatic, for example, the substituents may be phenyl, alkyl, alkenyl (such as vinyl), etc.

In the present application, unless otherwise specified, the term "aryl" refers to aromatic hydrocarbyl derived from the aromatic-ring hydrocarbon compound by losing a hydrogen atom on the aromatic ring, that is, a monovalent connection site is directly formed on the aromatic ring. The aryl may be monocyclic aryl, fused-ring aryl, or polycyclic aryl. For polycyclic ring species, at least one is an aromatic ring system. For example, "C₆-C₁₀ aryl" or "C₆₋₁₀ aryl" refers to aryl containing 6 to 10 carbon atoms, and may be independently C₆ aryl, C₈ aryl, C₉ aryl or C₁₀ aryl each time it appears. For aother example, "C₆-C₂₀ aryl" or "C₆₋₂₀ aryl" refers to aryl containing 6 to 20 carbon atoms, and may be independently but not limited to C₆ aryl aryl (such as phenyl),C₆ aryl aryl (such as benzocyclobutenyl),C₈ aryl (such as phenylpropylcyclobutenyl),C₉ aryl (such as indenyl),C₁₀ aryl (such as naphthyl),C₁₂ aryl (such as acenaphthenyl and biphenyl),C₁₃ aryl (such as fluorenyl),C₁₄ aryl (such as anthryl and phenanthryl),C₁₈ aryl (such as triphenylene) or C₂₀ aryl (such as perylenyl) each time it appears. Suitable examples of the aromatic-ring hydrocarbon compound include, but are not limited to, benzene, toluene, benzocyclobutene, biphenyl, indene, naphthalene, acenaphthene, fluorene, anthracene, phenanthrene, triphenylene, perylene, and derivatives thereof.

In the present application, unless otherwise specified, "heteroatom" refers to non-hydrogen and non-carbon atoms, such as O, N, S, P and B.

In the present application, unless otherwise specified, "heterohydrocarbyl" means that on the basis of hydrocarbyl, at least one carbon atom is replaced by a heteroatom, and the heteroatom may be but is not limited to an N atom, an O atom, an S atom, a P atom, a B atom, etc.

There is still a certain distance to go before the commercial large-scale application of perovskite cells, wherein energy conversion efficiency of perovskite thin films is one of the important factors restricting the industrialization of the perovskite cells. Furthermore, the uniformity of the perovskite thin films is one of the important factors in improving the energy conversion efficiency. To improve the uniformity of the perovskite thin films, certain surfactants may be added. However, if the added surfactant is inappropriate, it may cause the surfactants to form defects in perovskite crystals, resulting in deterioration of the performance of perovskite solar cells.

In response to the above common technical problems, **in** a first aspect, the present application provides a perovskite precursor solution, including a surfactant, a structure of the surfactant including a hydrophilic group, a hydrophobic group and a first functional group, wherein the first functional group is a Lewis base group containing a lone pair, and the lone pair is present in at least one of an N atom and an S atom; and the first functional group, the hydrophilic group and the hydrophobic group are different from each other.

In the present application, unless otherwise specified, the "hydrophilic group" and the "hydrophobic group" have the commonly known meanings in the art. Generally, the "hydrophilic group" refers to a group that is soluble in water or easily has affinity with water, and the "hydrophobic group" refers to a group that has no affinity for water, is insoluble in water, or has extremely low water solubility. It may be understood that the "hydrophilic group" and the "hydrophobic group" are relative. Non-limiting examples of the hydrophobic group applicable to the present application may include medium- and long-chain hydrocarbyl (such as C₈₋₂₀hydrocarbyl), C₇₋₂₅ aromatic hydrocarbyl, and the like. In the present application, some groups that are more hydrophilic than long-chain alkyl can be used as the "hydrophilic group" herein. Non-limiting examples of the hydrophilic group applicable to the present application may include one or more of proton acid, proton base, an ether group (-O-), an ester group (-COO-), an amide group (-CONH-), etc.; combined with an acid-base proton theory (Brønsted-Lowry acid-base theory), examples of the proton acid include a carboxyl group, a sulfonic acid group, a sulfonate salt, a sulfuric acid group, a phosphoric acid group, etc., as well as a group that complex organic or inorganic acid molecules such as carboxylic acid, phosphonic acid, sulfonic acid, hydrohalic acid, sulfuric acid, phosphoric acid, etc., and examples of the proton base include hydrophilic group, a primary amino group, a secondary amino group, a quaternary ammonium salt, etc.

In the present application, "lone pair" has a well-known meaning in the art, and refers to the presence of non-bonding electrons that are not used to form a covalent bond, "lone" means not forming a bond, and "bond" means that two electrons with opposite spins will pair up. For example, two nitrogen atoms in -NH-NH- and -NH-NH₂, and a sulfur atom in -SH, all have the lone pair.

In the present application, unless otherwise specified, "Lewis base" has a well-known meaning in the art, generally refers to base defined according to the acid-base electron theory of Gilbert Newton Lewis, and more specifically, refers to a substance that can provide an electron pair, including ions, atomic groups or molecules.

In the present application, unless otherwise specified, a "Lewis base group" refers to a group that can provide an electron pair.

In the present application, unless otherwise specified, a "Lewis base group containing a lone pair" means that the group contains an atom with the lone pair and the group can donate an electron pair.

In some embodiments, the present application provides a perovskite precursor solution, including a perovskite precursor material, a solvent and a surfactant; a structure of the surfactant includes a hydrophilic group, a hydrophobic group and a first functional group, wherein the first functional group is a Lewis base group containing a lone pair, and the lone pair is present in at least one of an N atom and an S atom; and the first functional group, the hydrophilic group and the hydrophobic group are different from each other.

In the structure of the surfactant having the hydrophilic group and the hydrophobic group, the lone pair may be provided by a specific type of atom (including at least one specific atom of N and S), so that the surfactant has suitable Lewis base property. On the one hand, wettability of the perovskite precursor solution on asubstrate surface can be improved, and on the other hand, the surfactant can more stably complex with a divalent metal ion in the perovskite precursor material and better passivate multiple defects, such as lead vacancy and iodine vacancy in perovskite. By introducing this surfactant of the special structure into the perovskite precursor solution, the uniformity of the perovskite thin film can be improved, and the energy conversion efficiency of the cell can be improved.

In addition, the perovskite precursor solution provided in the present application is advantageous for preparing a large-area (e.g. ≥ 1cm²) perovskite thin film.

In some embodiments, the first functional group includes one or more of -NR¹¹-NR¹²R¹³ and - SH; wherein R¹¹ and R¹² are each independently H or hydrocarbyl; R¹³ is H, hydrocarbyl, or hydrocarbyl substituted with a monovalent hydrophilic group; and the monovalent hydrophilic group may be selected from any suitable monovalent hydrophilic group herein. Examples of the monovalent hydrophilic group may include, but are not limited to, one or more of: carboxyl, a sulfonic acid group, a sulfonate salt, a sulfuric acid group, a sulfate salt, a phosphoric acid group, a phosphate salt, a monohydrogen phosphate group, a monohydrogen phosphate salt, a primary amino group, a primary amino salt, a secondary amino group, a secondary amino salt, a quaternary ammonium salt, -CONH₂, and hydroxyl.

In some embodiments, R¹¹ and R¹² are each independently H or C₁₋₂₀ hydrocarbyl, further optionally H or C₁₋₁₈ hydrocarbyl, still further optionally H or a C₁₋₁₃ hydrocarbyl, still further optionally H or C₁₋₁₂ hydrocarbyl, still further optionally H or C₁₋₁₀ hydrocarbyl, still further optionally H or a C₁₋₈ hydrocarbyl, still further optionally H or C₁₋₆ hydrocarbyl, still further optionally H or C₁₋₄ hydrocarbyl, still further optionally H or C₁₋₃ hydrocarbyl, still further optionally H or methyl.

In some embodiments, R¹³ is H, hydrocarbyl or hydrocarbyl substituted with a monovalent hydrophilic group; the hydrocarbyl in the hydrocarbyl or the hydrocarbyl substituted with the monovalent hydrophilic group may be each independently C₁₋₂₀ hydrocarbyl, optionally C₁₋₁₈ hydrocarbyl, further optionally C₁₋₁₃ hydrocarbyl, still further optionally C₁₋₁₂ hydrocarbyl, still further optionally C₁₋₁₀ hydrocarbyl, still further optionally C₁₋₈ hydrocarbyl, still further optionally C₁₋₆ hydrocarbyl, still further optionally C₁₋₄ hydrocarbyl, still further optionally C₁₋₃ hydrocarbyl, still further optionally methyl. The monovalent hydrophilic group may be defined as described above.

In some embodiments, R¹¹ and R¹² are each independently H or alkyl, optionally, R¹¹ and R¹² are each independently H or C₁₋₆alkyl, and further optionally, R¹¹ and R¹² are each independently H or C₁₋₃alkyl; still further optionally, R¹¹ and R¹² are each independently H or methyl; still further optionally, R¹¹ and R¹² are both H; still further optionally, R¹¹ and R¹² are both H; and still further optionally, the first functional group is selected from one or both of -NH-NH₂ and -SH.

In some embodiments, R¹³ may be H, alkyl or alkyl substituted with a hydrophilic group; the alkyl or the alkyl substituted with the hydrophilic group may be each independently C₁₋₂₀ alkyl, optionally C₁₋₁₈ alkyl, further optionally C₁₋₁₃ alkyl, still further optionally C₁₋₁₂alkyl, still further optionally C₁₋₁₀ alkyl, still further optionally C₁₋₈ alkyl, still further optionally C₁₋₆ alkyl, still further optionally C₁₋₄ alkyl, still further optionally C₁₋₃ alkyl, still further optionally methyl. The monovalent hydrophilic group may be defined as described above.

In some embodiments, R¹³ is H or hydrocarbyl, further optionally H or alkyl, still further optionally H or C₁₋₂₀ alkyl, still further optionally H or C₁₋₁₈ alkyl, still further optionally H or C₁₋₁₅ alkyl, still further optionally H or C₁₋₁₂ alkyl, still further optionally H or C₁₋₁₀ alkyl, still further optionally H or C₁₋₈ alkyl, still further optionally H or C₁₋₆ alkyl, still further optionally H or C₁₋₄ alkyl, still further optionally H or C₁₋₃ alkyl, still further optionally H or methyl. In some embodiments, R¹³ is H. In some embodiments, R¹³ is hydrocarbyl, which may also refer to the aforementioned definition.

In some embodiments, the first functional group includes one or more of -NR¹¹-NHR¹³ and - SH, R¹¹ and R¹³ are each independently H or hydrocarbyl, optionally, R¹¹ and R¹³ are each independently H or alkyl, further optionally, R¹¹ and R¹³ are each independently H or C₁₋₆ alkyl, still further optionally, R¹¹ and R¹³ are each independently H or C₁₋₃, still further optionally, R¹¹ and R¹³ are each independently H or methyl.

In some embodiments, the first functional group includes one or more of -NH-NHR¹³ and -SH, R¹³ may be H or hydrocarbyl, and R¹³ may also refer to the aforementioned definition.

A group having at least one of an N-N structure and -SH structure can be used as the first functional group, at this moment, more suitable Lewis base property can be provided, thereby playing a better role in improving the wettability of the perovskite precursor solution and reducing the vacancy defects in the perovskite, thereby better improving the uniformity of the perovskite thin film, which is also more beneficial to improving the energy conversion efficiency of the cell.

When the surfactant contains a -N-N- structure, it may be monovalent hydrazino or divalent hydrazino. The two nitrogen atoms in the -N-N- structure can both provide one lone pair. Taking a perovskite metal halide ABX₃ in the perovskite precursor material, where B is Pb²⁺ and X is I⁻ as an example, this type of surfactant containing the -N-N- structure may further provide a certain reducing property while serving as the Lewis base, which can reduce an iodine element, and inhibit oxidation of iodine ions, thereby reducing or avoiding the imbalance of a stoichiometric ratio and defects caused by it. The monovalent hydrazino is located at an end of a chain, and the divalent hydrazino is located in the middle of the chain, wherein the monovalent hydrazino at the end of the chain is easier to interact with Pb²⁺, and a passivation effect is better.

When the surfactant contains -SH, the sulfur atom can provide two lone pairs. This type of surfactant has stronger reducing property and interacts better with the perovskite components, which can promote uniform film formation of the perovskite solution while increasing the crystallization growth rate, promoting grain growth, and optimizing the bulk phase crystallization quality.

In the surfactant, when the types of the first functional groups in one molecule are greater than or equal to two, the number of any type of first functional group may independently be one or more (for the case of "more than one", such as ≥2, further such as 2 to 8, still further such as 2 to 5, still further such as 2, 3 or 4).

In some embodiments, the hydrophilic group includes one or more of carboxyl, a sulfonic acid group, a sulfonate salt, a sulfuric acid group, a sulfate salt, a phosphoric acid group, a phosphate salt, a monohydrogen phosphate group, a monohydrogen phosphate salt, a primary amino group, a primary amino salt, a secondary amino group, a secondary amino salt, a divalent tertiary amino group, a quaternary ammonium salt, -CONH₂, hydroxyl, an ether group, an ester group, -CONH- and a divalent phosphate ester group.

In the present application, unless otherwise specified, for the hydrophilic groups listed above, carboxyl is -COOH; the sulfonic acid group is -SO₃H, -S(=O)₂OH; the sulfonate salt may be - SO₃M, where M is an alkali metal ion, such as lithium, sodium, potassium, and further such as sodium and potassium; the sulfuric acid group is -O-S(=O)₂OH; the sulfate salt may be -O-S(=O)₂OM, where M is an alkali metal ion, such as lithium, sodium, potassium, and further such as sodium and potassium; the phosphate group is -OP(=O)(OH)₂; the phosphate salt may be a salt formed by replacing at least one hydrogen in -OP(=O)(OH)₂ with an alkali metal ion; the monohydrogen phosphate group may be a group formed by replacing one H in -OP(=O)(OH)₂ with hydrocarbyl or substituted hydrocarbyl, and may further be a group formed by replacing one H with alkyl or substituted alkyl, and the substituted hydrocarbyl or the substituted alkyl may each independently contain a heteroatom, and may further each independently contain any suitable hydrophilic group in the present application; the monohydrogen phosphate salt may be a salt formed by replacing one H in -OP(=O)(OH)₂ with hydrocarbyl or substituted hydrocarbyl and the other H with an alkali metal ion; the primary amino group is -NH₂; the primary amino salt is a group formed by complexation of -NH₂ with an acid molecule; the secondary amino group has a structure of >NH (or denoted as -NH-), and may be -NHR₀, R₀ is hydrocarbyl, optionally alkyl, further optionally C₁₋₃ alkyl, astill further optionally methyl; the secondary amino salt is a group formed by complexation of the secondary amino group with an acid molecule; the divalent tertiary amino is -NR₀-, and the definition of R₀ may be the same as that in the secondary amino group; the quaternary ammonium salt has a structure of>N⁺< and connects four carbon atoms, and may be *-N⁺R₀₁R₀₂R₀₃, where R₀₁; is alkyl; R₀₂ is hydrocarbyl, R₀₃ is alkyl; -CONH₂ is a monovalent amide group; the hydroxyl is -OH; the ether group is -O-; the ester group is -CO-O- or -O-CO-; -CONH-is a divalent amide group; the divalent phosphate ester group may be -O-(O=)P(OM₀₁)-O-, M₀₁is absent or is H or an alkali metal ion. Unless otherwise stated, one or both covalent connection sites of the hydrophilic group listed here are connected to a carbon atom.

In some embodiments, the hydrophilic group includes one or more of carboxyl, a sulfonic acid group, a sulfonate salt, a sulfuric acid group, a sulfate salt, a phosphoric acid group, a phosphate salt, a monohydrogen phosphate group, a monohydrogen phosphate salt, a primary amino group, a primary amino salt, a secondary amino group, a secondary amino salt, a quaternary ammonium salt, -CONH₂, and hydroxyl.

In some embodiments, the hydrophilic group includes one or more of carboxyl, a sulfonic acid group, a sulfonate salt, a sulfuric acid group, a sulfate salt, a phosphoric acid group, a phosphate salt, a primary amino group, a primary amino salt, a secondary amino group, a secondary amino salt, a quaternary ammonium salt, -CONH₂, and hydroxyl.

In some embodiments, the sulfonate salt, the sulfate salt, the phosphate salt and the monohydrogen phosphate salt are alkali metal salts of corresponding acids.

In some embodiments, the primary amino salt and the secondary amino salt are salts formed by corresponding amine and acid; optionally, the acid is organic acid or inorganic acid; the organic acid includes one or more of carboxylic acid, phosphonic acid, and sulfonic acid; the inorganic acid includes one or more of hydrohalic acid, phosphoric acid, and sulfuric acid; and optionally, the inorganic acid includes one or more of hydrochloric acid, hydroiodic acid, hydrobromic acid, hydrofluoric acid, and sulfuric acid.

In some embodiments, the hydrophilic group includes at least one of quaternary ammonium ions and alkali metal ions.

The surfactant provided in the present application may contain various types of hydrophilic groups, so that surface tension of the surfactant can be more flexibly adjusted, and the wettability of the perovskite precursor solution and the passivation effect on the vacancy defects in the perovskite can be more flexibly exerted. When the structure of the surfactant includes an alkali metal salt hydrophilic group, it is beneficial to further passivate interface defects of a perovskite layer. When the structure of the surfactant includes at least one of a primary amino group, a secondary amino group or tertiary aminoin, it is beneficial to passivate bulk phase defects. When the structure of the surfactant includes a quaternary ammonium salt, it is beneficial to control a nucleation rate and promote grain growth. When acid molecules are complexed in the structure of the surfactant, it is beneficial to the uniformity of large-area film formation of the perovskite layer.

The hydrophilic group can be not only a monovalent hydrophilic group located at an end group, but also a multivalent group as a linker, and can also be a combination of monovalent or multivalent hydrophilic groups, so that the hydrophilic group can be coordinated with the hydrophobic group and the first functional group by adjusting a position, number and other parameters of the hydrophilic group, thereby giving the perovskite precursor solution good wettability, reducing the vacancy defects in the perovskite, improving the uniformity of the perovskite thin film, and further improving the energy conversion efficiency of the cell.

In some embodiments, the hydrophilic group includes one or more of *-COOH, *-S(=O)₂OH, *-S(=O)₂OM, *-O-S(=O)₂OH, *-O-S(=O)₂OM, *-O-(O=)P(OH)₂, 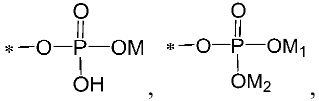 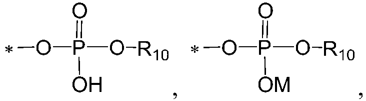 *-NH₂, *-NH₂·n₂A_{cd}, *-NHR₀, *-NHR₀·n₁A_{cd}, 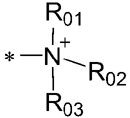 (e.g. 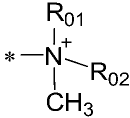 ), *-CONH₂, *-OH and a hydrophilic linker L₀; wherein the hydrophilic linker L₀ includes one or more of *-CONH-*, *-NHCO-*, *-C(=O)O-*, *-O-C(=O)-*, *-O-(O=)P(OM₀₁)-O-*, *-NH-* 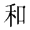 *-O-*;
wherein any "*" indicates a connection site to a carbon atom;
any M is independently an alkali metal ion (may be independently lithium, sodium or potassium, and may further be independently sodium or potassium);
any M₁ and any M₂ are each independently an alkali metal ion (may be each independently lithium, sodium or potassium, and may further be each independently sodium or potassium);
any R₁₀ is independently hydrocarbyl (may be independently C₁₋₁₀ alkyl, may further be independently C₁₋₈ alkyl, may still further be independently C₁₋₆ alkyl, may still further be independently C₁₋₄ alkyl, may still further be independently C₁₋₃ alkyl, and may still further be independently methyl, ethyl, or propyl);
any A_{cd} is independently an acid moledule (any A_{cd} may be independently an organic acid or inorganic acid molecule; the organic acid may include one or more of carboxylic acid, phosphonic acid, and sulfonic acid; the inorganic acid includes one or more of hydrohalic acid, phospronic acid and sulfuric acid; and optionally, the inorganic acid includes one or more of hydrochloric acid, hydroiodic acid, hydrobromic acid, hydrofluoric acid, and sulfuric acid); n₂ is 1 or 2; and n₁ is 1;
any R₀ is independently hydrocarbyl or substituted hydrocarbyl; the substituted hydrocarbyl is replaced by one or more hydrophilic groups; optionally, any R₀ is independently alkyl; further optionally, any R₀ is independently C₁₋₃ alkyl; still further optionally, R₀ is methyl;
R₀₁; is alkyl (optionally, R₀₁; is C₁₋₃ alkyl; further optionally, R₀₁ is methyl);
R₀₂ is hydrocarbyl (optionally, R₀₂ is C₁₋₈ alkyl or C₁₋₃ alkylene substituted with a benzene ring, and the benzene ring is phenyl or phenyl substituted with 1-4 C₁₋₃ alkyl; further optionally, R₀₂ is methyl or benzyl);
R₀₃ is alkyl (optionally, R₀₃ is C₁₋₃ alkyl; further optionally, R₀₃ is methyl);
M₀₁ is absent or is H or an alkali metal ion (optionally, M₀₁ is absent or is H, lithium, sodium or potassium, further optionally, M₀₁ is absent or is H, sodium or potassium). In some embodiments, M₀₁ is absent, in which case the O therein exists in a form of a negative ion (*-O-(O=)P(O⁻)-O-*). In some embodiments, M₀₁ is H or an alkali metal ion, may further be H, lithium, sodium or potassium, and may still further be H, sodium or potassium.

When the hydrophilic group includes a plurality of the aforementioned listed groups, different hydrophilic groups may be connected to different carbon atoms respectively, or two hydrophilic groups may be connected and combined to form a new hydrophilic group. For example, 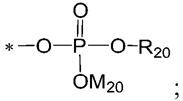 wherein the definition of M₂₀ may refer to but is not limited to M₀₁; M₂₀ may be absent or be H or an alkali metal ion (the alkali metal ion is optionally lithium, sodium or potassium, and is further optionally sodium or potassium); R₂₀ may be alkyl substituted with a hydrophilic group; optionally, R₂₀ is alkyl substituted with a quaternary ammonium salt type group; further optionally, R₂₀ is alkyl substituted with choline; still further optionally, R₂₀ is alkyl substituted with acetylcholine; in addition, the alkyl in the alkyl substituted with the hydrophilic group is optionally C₁₋₁₀alkyl, further optionally C₁₋₈ alkyl, still further optionally C₁₋₆ alkyl, still further optionally C₁₋₄ alkyl, still further optionally C₁₋₃ alkyl. For another example, *-NHR₃₀, where R₃₀ may be alkyl substituted with one or more hydrophilic groups; optionally, R₃₀ is C₁₋₃ alkyl substituted with one or more hydrophilic groups.

In some embodiments, the monovalent hydrophilic group is connected to at least one side of the hydrophilic linker L₀.

In some embodiments, the surfactant satisfies any one or more of the following features:
any M is independently lithium, sodium or potassium;
any M₁ and any M₂ are each independently lithium, sodium or potassium;
any R₁₀ is independently C₁₋₁₀ alkyl, optionally C₁₋₈ alkyl, further optionally C₁₋₆ alkyl, still further optionally C₁₋₄ alkyl, still further optionally C₁₋₃ alkyl;
any A_{cd} is independently an organic acid or inorganic acid molecule; the organic acid includes one or more of carboxylic acid, phosphonic acid, and sulfonic acid; the inorganic acid includes one or more of hydrohalic acid, phosphoric acid and sulfuric acid; and optionally, the inorganic acid includes one or more of hydrochloric acid, hydroiodic acid, hydrobromic acid, hydrofluoric acid, and sulfuric acid;
any R₀ is independently alkyl; optionally, any R₀ is independently C₁₋₃ alkyl; further optionally, R₀ is methyl;
R₀₁; is C₁₋₃ alkyl; optionally, R₀₁; is methyl;
R₀₂ is C₁₋₈ alkyl or C₁₋₃ alkylene substituted with a benzene ring, and the benzene ring is phenyl or phenyl substituted with 1-4 C₁₋₃ alkyl; optionally, R₀₂ is methyl or benzyl;
R₀₃ is C₁₋₃ alkyl; optionally, R₀₃ is methyl;
M₀₁ is absent or is H, lithium, sodium or potassium; and
the monovalent hydrophilic group is connected to at least one side of the hydrophilic linker L₀.

In some embodiments, the hydrophobic group includes a carbon chain, and further includes a C₈₋₂₀ carbon chain.

In the present application, unless otherwise specified, the "carbon-chain" refers to a monovalent group having a plurality of carbon atoms linearly connected in sequence from the connecting site to the farthest end group, or a multivalent group connected to a plurality of connection sites through the plurality of carbon atoms linearly connected in sequence (e.g., a divalent group connected to two connection sites). Those carbon atoms connected sequentially along the longest interval constitute main chain carbon atoms of the carbon chain. For example, the number of the main chain carbon atoms of the trivalent group -CH₂CH₂CH(CH₂-)-CH₂CH₂CH₂CH₂- is 7, and the number of main chain carbon atoms of the trivalent group - CH₂CH(CH₂CH₂CH₂-)-CH₂CH₂CH₂CH₂- is 8. It should be noted that the hydrogen atoms on these main chain carbon atoms may be substituted by hydrocarbyl or heterohydrocarbyl. When substituted by the heterohydrocarbyl, the heteroatoms may be present in the form of, but not limited to, the aforementioned hydrophilic group.

In the present application, unless otherwise specified, the connection site of the carbon chain in the surfactant is connected to the heteroatom in the surfactant, or forms a heteroatom-containing connection group with the adjacent atom or atom group, such as an ester group (-CO-O- or -O-CO-), an amide group (-CO-NH- or -NH-CO-), an ether bond (-O-), a secondary amino group (-NH-), divalent tertiary amino (-N(CH₃)-), and the like.

In the present application, the C₈₋₂₀ carbon chain refers to a carbon chain having 8-20 carbon atoms. The number of carbon atoms in the C₈₋₂₀ carbon chain may also be 8, 9, 10, 11, 12, 13, 14, 15, 16, 17, 18, 19 or 20, or may be an interval consisting of any two of the aforementioned values, such as 8-18, 10~18, and 12~18. Non-limiting examples of the carbon chain are octyl, nonyl, decyl, undecyl, dodecyl, tridecyl, tetradecyl, pentadecyl, hexadecyl, heptadecyl, octadecyl, nonadecyl, eicosyl, octylene, nonylene, decylene, divalent undecyl, divalent dodecyl, divalent tridecyl, divalent tetradecyl, divalent pentadecyl, divalent hexadecyl, divalent heptadecyl, divalent octadecyl, divalent nonadecyl, divalent eicosyl, or an unsaturated form of any of the foregoing groups, or any of the foregoing containing or unsaturated structures in which at least one hydrogen is substituted by hydrocarbyl or heterohydrocarbyl.

By introducing the hydrophobic group of a certain size into the structure of the surfactant, the hydrophobic group can cooperate with the hydrophilic group and the first functional group to form a hydrophilic-hydrophobic balance that is more suitable for the perovskite precursor solution of the present application. While playing the role of the lone pair in the first functional group, a perovskite precursor solution system can be made more stable and better dispersed, with better wettability to the substrate, thereby better improving the uniformity of the perovskite thin film.

In some embodiments, the carbon chain in the hydrophobic group is a linear or branched structure. In some embodiments, the C₈₋₂₀ carbon chain in the hydrophobic group is a linear or branched structure. At this time, the C₈₋₂₀ carbon chain does not contain a ring structure, and the molecular flexibility is good, which can adjust the flexibility of the perovskite thin film.

In some embodiments, the main chain atom length of the C₈₋₂₀ carbon chain in the hydrophobic group is in a range from 6 to 20; further optionally, the main chain atom length of the C₈₋₂₀ carbon chain is in a range from 8 to 20; still further optionally, the main chain atom length of the C₈₋₂₀ carbon chain is in a range from 8 to 18; and still further optionally, the C₈₋₂₀ carbon chain in the hydrophobic group is a C₁₀₋₂₀ carbon chain with the main chain atom length of 10 to 18. The main chain atom length of the C₈₋₂₀ carbon chain may be 6, 7, 8, 9, 10, 11, 12, 13, 14, 15, 16, 17, 18, 19 or 20, or may be an interval consisting of any two of the aforementioned lengths. It may be understood that the number of carbon atoms in the C₈₋₂₀ carbon chain is greater than or equal to the main chain atom length.

In the present application, unless otherwise specified, the "main chain atom length of the carbon chain" refers to a length of a spacer atom connecting two designated sites (one of which is a connection site and the other is a connection site or endpoint), and the spacer atom length refers to the number of carbon atoms connected sequentially between the two connection sites; for a polyvalent carbon chain containing three or more connection sites, the main chain atom length is the number of carbon atoms connected sequentially between the two connection sites with the farthest interval. For example, the spacer atom length of 1,3-propylidene is 3, the spacer atom length of 1,2-propylidene is 2, the spacer atom length of the divalent group -CH(CH₂CH₃)- is 1, the spacer atom length of the trivalent group -CH₂CH₂CH(CH₂-)-CH₂CH₂CH₂CH₂- is 7 (corresponding to 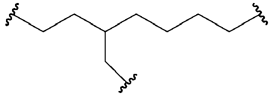 ), and the spacer atom length of the trivalent group - CH₂CH(CH₂CH₂CH₂-)-CH₂CH₂CH₂CH₂- is 8.

In some embodiments, the C₈₋₂₀ carbon chain is a saturated structure or an unsaturated structure. Non-limiting examples of the saturated structure are octyl, nonyl, decyl, undecyl, dodecyl, tridecyl, tetradecyl, pentadecyl, hexadecyl, heptadecyl, octadecyl, nonadecyl, eicosyl, octylene, nonylene, decylene, divalent undecyl, divalent dodecyl, divalent tridecyl, divalent tetradecyl, divalent pentadecyl, divalent hexadecyl, divalent heptadecyl, divalent octadecyl, divalent nonadecyl, and divalent eicosyl, and may further be a linear chain form of any of the aforementioned groups, such as monovalent -(CH₂)₉CH₃, divalent -(CH₂)₁₀-, monovalent -(CH₂)₁₁CH₃, divalent -(CH₂)₁₂, monovalent -(CH₂)₁₃CH₃, divalent -(CH₂)₁₄-, monovalent -(CH₂)₁₅CH₃, divalent -(CH₂)₁₆-, monovalent -(CH₂)₁₇CH₃, divalent -(CH₂)₁₈-, and the like. Non-limiting examples of the unsaturated structure are unsaturated forms of the aforementioned saturated structure, which may include one or more (such as 2 or 3, further such as 2) carbon-carbon unsaturated bonds, and further, the carbon-carbon unsaturated bonds may be, but are not limited to alkenyl or alkynyl. Non-limiting examples of the unsaturated structure are further CH₃-(CH₂)₄-CH=CHCH₂CH=CH-(CH₂)₇C(=O)-, in which case the connection site on the right side is substituted with oxygen, the main chain atom length of the carbon chain is 18, and it is an unsaturated form containing two unsaturated carbon-carbon double bonds.

For another example, 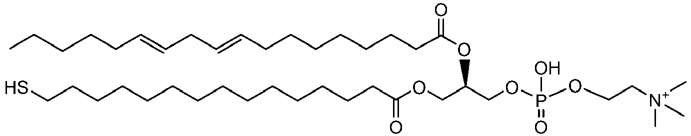 includes at least the following carbon chains: a monovalent carbon chain CH₃-(CH₂)₄-CH=CHCH₂CH=CH-(CH₂)₇C(=O)- and a divalent carbon chain -(CH₂)₁₄C(=O)-; it can also be considered that the surfactant includes at least the following carbon chains: a monovalent carbon chain CH₃-(CH₂)₄-CH=CHCH₂CH=CH-(CH₂)₇- and a divalent carbon chain -(CH₂)₁₄-, in which case the two longer carbon chains are respectively connected to the carbon atoms in the divalent linker ester group -C(=O)-O-.

In some embodiments, the C₈₋₂₀ carbon chain is an aromatic chain or an aliphatic chain.

In the present application, unless otherwise specified, the "aromatic chain" includes at least one aromatic ring. The aliphatic chain do not contain any aromatic ring. Those skilled in the art are aware of the meaning of the "aromatic ring", and the aromatic ring refers to a ring with an aromatic property, and may be an aromatic ring or a heteroaromatic ring. Ring atoms of the "aromatic ring" are all carbon atoms. Ring atoms of the heteroaromatic ring include at least one heteroatom. The valence of the aromatic ring is not particularly limited, and may be monovalent or polyvalent, such as monovalent or divalent. For the aromatic chain, the aromatic ring may be used to provide main chain atoms (such as -CH₂-Ph-CH₂-), or may be used as a substituent without participating in the main chain composition (such as -CH₂-CH(Ph)-CH₂-, -CH₂-CH(pyridyl)-CH₂-). Ph represents a benzene ring.

In some implementations, the molecular structure of the surfactant includes one or more C₈₋₂₀ carbon chains. In a molecular structure of the surfactant, the number of the C₈₋₂₀ carbon chains may be one or more, such as 1 or 2.

In some embodiments, the hydrophobic group includes one or more of arylene Ar₀ and arylalkyl ArA.

In some embodiments, the hydrophobic group includes arylene Ar₀.

In some embodiments, the hydrophobic group includes arylalkyl ArA.

In the present application, unless otherwise specified, the "arylene" refers to divalent aromatic hydrocarbyl derived from an aromatic-ring hydrocarbon compound by losing two hydrogen atoms on the aromatic ring, that is, two monovalent connection sites are directly formed on the ring, or the "arylene" refers to divalent aromatic hydrocarbyl formed by losing another hydrogen atom on the aromatic ring based on aryl. The aromatic ring of the arylene may or may not have a substituent. When the substituent is present, the substituent may be aromatic or non-aromatic. It may be understood that the substituent on the aromatic ring should also belong to hydrocarbyl. For example, "C₆₋₁₂ arylene" refers to that the group has 6 to 12 carbon atoms, which may independently be C₆ arylene, C₇ arylene, C₈ arylene, C₉ arylene, C₁₀ arylene, C₁ arylene, and C₁₂ arylene each time it appears. Suitable examples include, but are not limited to, phenylene, 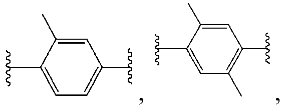 naphthylene, and the like. In the present application, unless otherwise specified, the " 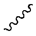 " at the terminal in the structural formula represents the connection site of the covalent bond.

In the present application, unless otherwise specified, "aralkane" refers to a hydrocarbon compound formed by replacing one or more hydrogen atoms on the aromatic ring with alkyl; further, unless otherwise specified, it generally refers to hydrocarbon formed by replacing one hydrogen atom on the aromatic ring with alkyl. Unless otherwise specified, "aralkyl" refers to hydrocarbyl derived from alkyl carbon in the corresponding aralkane by losing one hydrogen atom, that is, the aralkyl is introduced out of the connection center through the "alkyl" carbon.

In some embodiments, the arylene Ar₀ is C₆₋₁₈ arylene, may further be phenylene or phenylene substituted with one or more C₁₋₃ alkyl, and may still further be phenylene or phenylene substituted with one or more methyl.

In some embodiments, the arylene Ar₀ is phenylene.

In some embodiments, the aralkyl ArA is C₁₋₁₈ alkyl substituted with one or more aryl Ar₁, wherein any one of the aryl Ar₁ is independently phenyl or phenyl substituted with one or more C₁₋₄ alkyl; further optionally, any one of the aryl Ar₁ is independently phenyl or phenyl substituted with one or more C₁₋₃ alkyl; and still further optionally, any one of the aryl Ar₁ is independently phenyl or benzyl.

In some embodiments, the aralkyl ArA is benzyl.

For example, 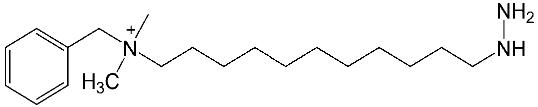 includes the following carbon chains: -(CH₂)₁₁- and benzyl. For example, 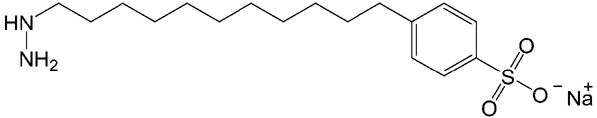 includes the following carbon chains: -(CH₂)₁₁- and phenylene.

The aromatic ring may be introduced into the hydrophobic structure of the surfactant, which is more conducive to the interaction between surfactant molecules and perovskite components, and can promote better dissolution of the surfactant in the perovskite precursor solution.

In some embodiments, in one molecule of the surfactant, the number of the hydrophilic groups, the hydrophobic groups and the first functional groups is each independently one or more.

In some embodiments, in one molecule of the surfactant, the number of the first functional groups is 1 or 2-5 (such as 1 or 2, 3, 4 or 5, further such as 1 or 2 or 3, still further such as 1 or 2).

In some embodiments, in one molecule of the surfactant, the number of the first functional groups may be 1 to 5, and further may be 1 to 3.
optionally, in one molecule of the surfactant, the number of the hydrophilic groups is 1 or 2-5 (such as 1 or 2, 3, 4 or 5, further such as 1 or 2 or 3, still further such as 1 or 2).

In some embodiments, in one molecule of the surfactant, the number of the hydrophilic groups is 1 to 5, and further may be 1 to 3.

The number of the hydrophilic groups, the hydrophobic groups and the first functional groups in the surfactant can be adjusted respectively, so that the surfactant can simultaneously obtain a suitable hydrophilic-hydrophobic balance and Lewis basicity of the lone pair in a wider range.

In some embodiments, in one molecule of the surfactant, the number of the hydrophilic groups is 1 to 5, and further may be 1 to 3.

In some embodiments, a molar percentage of the first functional group relative to the divalent metal ion in the perovskite precursor material is in a range from 0.001 mol% to 5 mol%. The molar percentage of the first functional group relative to the divalent metal ion in the perovskite precursor material may also be selected from any one of the following molar percentages: 0.001 mol%, 0.005 mol%, 0.01 mol%, 0.02 mol%, 0.04 mol%, 0.05 mol%, 0.06 mol%, 0.08 mol%, 0.1 mol%, 0.12 mol%, 0.14 mol%, 0.15 mol%, 0.16 mol%, 0.18 mol%, 0.2 mol%, 0.22 mol%, 0.24 mol%, 0.25 mol%, 0.26 mol%, 0.28 mol%, 0.3 mol%, 0.35 mol%, 0.4 mol%, 0.45 mol%, 0.5 mol%, 0.55 mol%, 0.6 mol%, 0.65mol%, 0.7 mol%, 0.75 mol%, 0.8 mol%, 0.9 mol%, 1 mol%, 1.2 mol%, 1.4 mol%, 1.5 mol%, 1.6 mol%, 1.8 mol%, 2 mol%, 2.5 mol%, 3 mol%, 3.5 mol%, 4 mol%, 4.5 mol%. 5 mol% and the like, and may further be selected from an interval composed of any two molar percentages above, such as 0.01 mol%-2.5 mol%, and 0.1 mol%-1 mol%.

The amount of the first functional group can be adjusted according to the usage amount of the divalent metal ion in the perovskite precursor material, so as to better realize the synergistic effect of the surfactant and the perovskite precursor material while both improving the wettability of the perovskite precursor solution and reducing the vacancy defects in the perovskite, thereby better improving the uniformity of the perovskite thin film, which is also more beneficial to improving the energy conversion efficiency of the cell.

In some embodiments, the first functional group and the hydrophilic group are each independently connected to a carbon atom of the hydrophobic group. "Each independently" means that they may be directly connected or indirectly connected via a linker.

In some embodiments, the surfactant includes one or more of the following compounds: 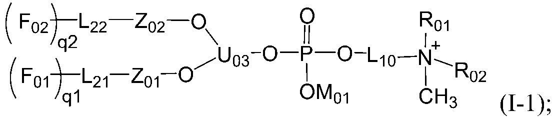 in formula (I-1), q1 and q2 are each independently 0 or a positive integer, q1+q2≥1, and L₂₁ and L₂₂ are each independently polyvalent hydrocarbyl having a main chain atom length of 8 to 20; Z₀₁; and Z₀₂ are each independently a covalent bond, carbonyl or -NHC(=O)-*, wherein * points to U₀₃; U₀₃ is trivalent hydrocarbyl; M₀₁is absent or is H or an alkali metal ion (also refer to the previous definition of M₀₁); L₁₀ is C₁₋₆ alkylene; R₀₁; is alkyl; R₀₂ is hydrocarbyl; in one molecule, at least one of F₀₁ and F₀₂ is the first functional group, and the other is independently H or the first functional group; 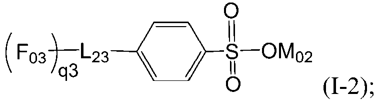 in formula (I-2), M₀₂ is an alkali metal ion; 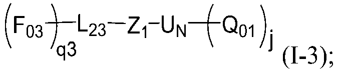 in formula (I-3), Z₁ is a covalent bond or a linker Z₁₀, wherein Z₁₀ is selected from any one of the following groups: -CO-NH, -NH-CO-, -C(=O)-O-, -O-C(=O)-, -NH-C(=O)-O-, -O-C(=O)-NH- and -O-; U_{N} is a j+1 valent group; j is a positive integer; any Q₀₁ is independently -OH or -COOM₀₃, M₀₃ is H or an alkali metal ion; 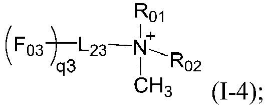 in formula (I-4), R₀₁ is alkyl; R₀₂ is hydrocarbyl;
in formula (I-2), formula (I-3), formula (I-4) and formula (I-5), F₀₃ is respectively and independently the first functional group; and
in formula (I-2), formula (I-3), formula (I-4) and formula (I-5), q3 is each independently an integer greater than 1, and L₂₃ is each independently polyvalent hydrocarbyl having a main chain atom length of 8 to 20.

In some embodiments, q1 and q2 are each independently 0 or a positive integer, and q1+q2≥1. Optionally, q1+q2 is an integer from 1 to 5 (such as 1, 2, 3, 4 or 5), and is further optionally 1 or 2. In some embodiments, q1 is 1 or 2, and q2 is 0. In some embodiments, q1 is 1, and q2 is 0. In some other embodiments, q1 is 2, and q2 is 0.

In some embodiments, q3 is 1 or an integer greater than or equal to 2, and is further optionally 1 or 2. In some embodiments, q3 is 1. In some other embodiments, q3 is 2.

In some embodiments, the surfactant includes a compound represented by formula (I-1), and may further be a compound represented by formula (I-1). The compound shown in (I-1) is easily soluble in the perovskite precursor solution, has little or no effect on the viscosity of the original solution, and is conducive to large-area film formation.

In some embodiments, the surfactant includes a compound represented by formula (I-2), and may further be a compound represented by formula (I-2). The compound represented by formula (I-2) can improve film formation while reducing the iodine element (taking X in ABX₃ being iodine as an example), filling vacancies, and effectively inhibiting the generation of perovskite phase defects.

In some embodiments, the surfactant includes a compound represented by formula (I-3), and may further be a compound represented by formula (I-3). The compound represented by formula (I-3) can improve solubility while filling the A-site defect, stabilizing the perovskite structure, and enhancing the stability of the thin film after crystallization.

In some embodiments, the surfactant includes a compound represented by formula (I-4), and may further be a compound represented by formula (I-4). The compound represented by formula (I-4) can promote film formation while reducing the number of defects at the interface and optimizing carrier transport at the interface.

For the surfactant containing the quaternary ammonium salt group, while adjusting film formation, the surfactant can interact with the intermediate phase of perovskite, so as to adjust the crystallization nucleation rate and crystallization rate, and promote grain growth.

For the surfactant containing a phosphate group, an interface between the perovskite layer and a hole transport layer can be passivated.

For the branched surfactant containing at least two hydrophobic carbon chains, the damage of water to the perovskite layer can be avoided.

For the linear surfactant containing sulfonate, bulk phase defects may be reduced and short-circuit current density may be increased.

For the surfactant whose hydrophilic group includes one or more hydroxyl, the solubility of the surfactant in the precursor solution can be increased, thereby improving the uniformity of large-area coating.

For the surfactant whose hydrophilic group includes one or more carboxyl, the bulk phase defects can be reduced, and device stability can be improved.

In some embodiments, in formula (I-1), L₂₁ and L₂₂ are each independently a linear or branched type, and may each independently be a saturated structure or an unsaturated structure; optionally, L₂₁ and L₂₂ are each independently linear C₈₋₂₀ alkylene or branched C₈₋₂₀ polyvalent hydrocarbyl; further optionally, L₂₁ and L₂₂ are each independently linear C₈₋₂₀ hydrocarbylene; still further optionally, L₂₁ and L₂₂ are each independently linear C₈₋₂₀ alkylene or linear C₈₋₂₀ alkenylene; still further optionally, L₂₁ and L₂₂ are each independently linear C₁₀₋₁₈ alkylene or linear C₁₀₋₁₈ alkenylene; still further optionally, L₂₁ and L₂₂ are each independently linear C₁₂₋₁₈ alkylene or linear C₁₂₋₁₈ alkenylene. Examples of the saturated structure of L₂₁ and L₂₂ are *-(CH₂)₉-*; and examples of the unsaturated structure are *-(CH₂)₄-CH=CHCH₂CH=CH-(CH₂)₇-*, where * indicates a connection site.

Non-limiting examples of the linear type L₂₁ or L₂₂ are nonylene *-(CH₂)₉-*, and * represent a connection site.

Non-limiting examples of branched L₂₁ or L₂₂ include trivalent hydrocarbyl *-(CH₂)₂-CH(-*)CH₂-CH=CHCH₂CH=CH-(CH₂)₇-*, and * represent a connection site. For another example, *-(CH₂)₂-CH₂CH₂-CH=C(-*)CH₂CH=CH-(CH₂)₇-*, and * represents a connection site.

In some embodiments, in formula (I-1), U₀₃ is trivalent alkyl or 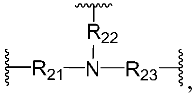 and R₂₁ R₂₂ and R₂₃ are each independently alkylene; optionally, U₀₃ is trivalent C₂₋₁₀ alkyl or trivalent C₃₋₁₀ tertiary amino, further optionally, U₀₃ is -CH₂-CR₀₄(-)-CH₂-, >CH-L₀₄- or N(-CH₂CH₂-)₃, R₀₄ is H or C₁₋₄ alkyl (R₀₄ is further optionally H, methyl or ethyl, still further optionally H), L₀₄ is C₁₋₆ alkylene (L₀₄ is further optionally C₁₋₄ alkylene, still further optionally C₁₋₄ alkylene, still further optionally methylene, 1,2-ethylidene, 1,3-propylidene or 1,4-butylidene); optionally, R₂₁ R₂₂ and R₂₃ are each independently C₁₋₄ alkylene, further optionally, R₂₁, R₂₂ and R₂₃ are each independently C₁₋₃ alkylene, still further optionally, R₂₁, R₂₂ and R₂₃ are each independently methylene or ethylidene, and still further optionally, R₂₁, R₂₂ and R₂₃ are all ethylidene.

In some embodiments, in formula (I-1), M₀₁ is absent or is H, a lithium ion, a sodium ion or a potassium ion.

In some embodiments, in formula (I-1), L₁₀ is C₁₋₄ alkylene, optionally ethylidene or propylidene, afurther optionally ethylidene.

In some embodiments, in formula (I-1), R₀₁; is C₁₋₃ alkyl; optionally, R₀₁ is methyl.

In some embodiments, in formula (I-1), R₀₁; is C₁₋₈ alkyl or C₁₋₃ alkylene substituted with a benzene ring, wherein the benzene ring is phenyl or phenyl substituted with 1-4 C₁₋₃ alkyl; optionally, R₀₂ is a methyl or benzyl.

In some embodiments, in formula (I-2), M₀₂ is H, a lithium ion, a sodium ion or a potassium ion.

In some embodiments, in formula (I-3), j is an integer selected from 1 to 5, optionally an integer selected from 1 to 4, further optionally 1, 2 or 3.

In some embodiments, R₀₁; is C₁₋₃ alkyl; optionally, R₀₁ is methyl.

In some embodiments, R₀₂ is C₁₋₈ alkyl or C₁₋₃ alkylene substituted with a benzene ring, wherein the benzene ring is phenyl or phenyl substituted with 1-4 C₁₋₃ alkyl; optionally, R₀₂ is a methyl or benzyl.

In some embodiments, in formula (I-3), U_{N} is polyvalent hydrocarbyl containing 0, 1 or more hydrophilic linkers L₀₁, optionally, U_{N} is polyvalent saturated hydrocarbyl containing 0, 1 or more hydrophilic linkers L₀₁, any hydrophilic linker L₀; is independently *-CONH-*, *-NHCO-*, *-C(=O)O-*, *-O-C(=O)-*, *-O-(O=)P(OM₀₁)-O-* or *-O-*, wherein * represents the connection site to the carbon atom, and M₀₁ is absent or is H, a lithium ion, a sodium ion or a potassium ion.

In some embodiments, in formula (I-3), U_{N} is trivalent alkyl or tetravalent alkyl. Optionally, the number of carbon atoms of U_{N} is 3 to 10, further optionally 3 to 6, further optionally 3, 4 or 5; still further optionally, U_{N} is -CH₂₋CR₀₅(-)-CH₂-, R₀₅ is H, methyl or ethyl; and still further optionally, R₀₅ is H.

In some embodiments, in formula (I-3), U_{N} is trivalent tertiary amino, optionally, trivalent C₃₋₁₀ tertiary amino, further optionally, N(-CH₂CH₂-)₃.

In some embodiments, in formula (I-3), the number of non-hydrogen atoms of U_{N} is in a range from 2 to 40, optionally in a range from 2 to 30, further optionally in a range from 2 to 25, still further optionally in a range from 2 to 20, still further optionally in a range from 2 to 18, still further optionally in a range from 2 to 15, still further optionally in a range from 2 to 12, still further optionally in a range from 2 to 10, still further optionally in a range from 2 to 8, still further optionally in a range from 2 to 6, still further optionally 2, 3, 4 or 5.

In some embodiments, in formula (I-3), j is an integer selected from 1 to 5, optionally an integer selected from 1 to 4, further optionally 1, 2 or 3.

In some embodiments, in formula (I-4), R₀₁; is C₁₋₃ alkyl; optionally, R₀₁ is methyl.

In some embodiments, in formula (I-4), R₀₁; is C₁₋₈ alkyl or C₁₋₃ alkylene substituted with a benzene ring, wherein the benzene ring is phenyl or phenyl substituted with 1-4 C₁₋₃ alkyl; optionally, R₀₂ is a methyl or benzyl.

In some embodiments, in formula (I-2), formula (I-3) and formula (I-4), L₂₃ is respectively and independently a linear or branched type, and may be a saturated structure or an unsaturated structure; optionally, L₂₃ is respectively and independently linear C₈₋₂₀ hydrocarbylene or branched C₈₋₂₀ polyvalent hydrocarbyl; further optionally, L₂₃ is respectively and independently linear C₈₋₂₀ alkylene or linear C₈₋₂₀ alkenylene; still further optionally, L₂₃ is respectively and independently linear C₁₀₋₁₈ alkylene or linear C₁₀₋₁₈ alkenylene; still further optionally, L₂₃ is respectively and independently linear C₁₂₋₁₈ alkylene or linear C₁₂₋₁₈ alkenylene. Examples of the saturated structure of L₂₃ are *-(CH₂)₉-*; and examples of the unsaturated structure are *-(CH₂)₄-CH=CHCH₂CH=CH-(CH₂)₇-*, where * is a connection site.

In the present application, unless otherwise specified, "alkenylene" refers to hydrocarbyl having two monovalent group centers derived from alkenyl by removing one hydrogen atom, and may be unsaturated branched hydrocarbyl or unsaturated linear hydrocarbyl. For example, "C₂₋₉ alkenylene" refers to an alkenyl moiety containing 2 to 9 carbon atoms, which may independently be C₂ alkenylene, C₄ alkylene, C₅ alkenylene, C₆ alkenylene, C₇ alkenylene, C₈ alkenylene, or C₉ alkenylene each time it appears. Suitable examples include, but are not limited to, 1,2-vinyl (-CH=CH-).

In the present application, the term "alkenyl" refers to a monovalent residue formed by a chain-like olefin compound losing one hydrogen atom, and the hydrogen atom may be located on a carbon-carbon double bond or on an alkyl substituent of the carbon-carbon double bond. Phrases containing this term, for example, "C₂₋₁₀ alkenyl" refers to alkenyl containing 2 to 10 carbon atoms, which may independently be C₂ alkenyl, C₃ alkenyl, C₄ alkenyl, C₅ alkenyl, C₆ alkenyl, C₇ alkenyl, C₈ alkenyl, C₉ alkenyl or C₁₀ alkenyl each time it appears. Suitable examples include, but are not limited to, vinyl (CH₂=CH-), allyl (CH₂=CH-CH₂-), CH₃-CH=CH-, and the like.

In some embodiments, the surfactant satisfies any one or more of the following features:

In formula (I-1), L₂₁ and L₂₂ are each independently a linear or branched type; optionally, L₂₁ and L₂₂ are each independently linear C₈₋₂₀ hydrocarbylene; further optionally, L₂₁ and L₂₂ are each independently linear C₈₋₂₀ alkylene or linear C₈₋₂₀ alkenylene; still further optionally, L₂₁ and L₂₂ are each independently linear C₁₀₋₁₈ alkylene or linear C₁₀₋₁₈ alkenylene; still further optionally, L₂₁ and L₂₂ are each independently linear C₁₂₋₁₈ alkylene or linear C₁₂₋₁₈ alkenylene;
in formula (I-1), U₀₃ is trivalent alkyl or 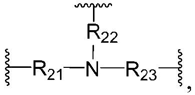 and R₂₁, R₂₂ and R₂₃ are each independently alkylene; optionally, U₀₃ is trivalent C₂₋₁₀ alkyl or trivalent C₃₋₁₀ tertiary amino, further optionally, U₀₃ is -CH₂-CR₀₄(-)-CH₂-, >CH-L₀₄- or N(-CH₂CH₂-)₃, R₀₄ is H or C₁₋₄ alkyl (R₀₄ is further optionally H, methyl or ethyl, still further optionally H), L₀₄ is C₁₋₆ alkylene (L₀₄ is further optionally C₁₋₄ alkylene, still further optionally C₁₋₄ alkylene, still further optionally methylene, 1,2-ethylidene, 1,3-propylidene or 1,4-butylidene); optionally, R₂₁, R₂₂ and R₂₃ are each independently C₁₋₄ alkylene, further optionally, R₂₁, R₂₂ and R₂₃ are each independently C₁₋₃ alkylene, still further optionally, R₂₁, R₂₂ and R₂₃ are each independently methylene or ethylidene, and still further optionally, R₂₁, R₂₂ and R₂₃ are all ethylidene;
in formula (I-1), M₀₁ is absent or is H, a lithium ion, a sodium ion or a potassium ion;
in formula (I-1), L₁₀ is C₁₋₄ alkylene, optionally ethylidene or propylidene, afurther optionally ethylidene;
in formula (I-1), R₀₁; is C₁₋₃ alkyl; optionally, R₀₁; is methyl;
in formula (I-1), R₀₁; is C₁₋₈ alkyl or C₁₋₃ alkylene substituted with a benzene ring, wherein the benzene ring is phenyl or phenyl substituted with 1-4 C₁₋₃ alkyl; optionally, R₀₂ is methyl or benzyl;
in formula (I-2), M₀₂ is H, a lithium ion, a sodium ion or a potassium ion;
n formula (I-3), U_{N} is polyvalent hydrocarbyl containing 0, 1 or more hydrophilic linkers L₀₁, optionally, U_{N} is polyvalent saturated hydrocarbyl containing 0, 1 or more hydrophilic linkers L₀₁, any of the hydrophilic linkers L₀₁; is independently *-CONH-*, *-NHCO-*, *-C(=O)O-*, *-O-C(=O)-*, *-O-(O=)P(OM₀₁)-O-* or *-O-*, wherein * represents a connection site to a carbon atom, and M₀₁ is absent or is H, a lithium ion, a sodium ion or a potassium ion; alternatively, U_{N} is trivalent alkyl or tetravalent alkyl, optionally, the number of carbon atoms of U_{N} is in a range from 3 to 10, further optionally in a range from 3 to 6, further optionally 3, 4 or 5, still further optionally, U_{N} is -CH₂₋CR₀₅(-)-CH₂-, R₀₅ is H, methyl or ethyl, and still further optionally, R₀₅ is H; alternatively, U_{N} is trivalent tertiary amino, optionally trivalent C₃₋₁₀tertiary amino, and further optionally N(-CH₂CH₂-)₃;
in formula (I-3), the number of non-hydrogen atoms of U_{N} is in a range from 2 to 40, optionally in a range from 2 to 30, further optionally in a range from 2 to 25, still further optionally in a range from 2 to 20, still further optionally in a range from 2 to 18, still further optionally in a range from 2 to 15, still further optionally in a range from 2 to 12, still further optionally in a range from 2 to 10, still further optionally in a range from 2 to 8, still further optionally in a range from 2 to 6, still further optionally 2, 3, 4 or 5;
in formula (I-3), j is an integer selected from 1 to 5, optionally an integer selected from 1 to 4, further optionally 1, 2 or 3;
in formula (I-4), R₀₁; is C₁₋₃ alkyl; optionally, R₀₁; is methyl;
in formula (1-4), R₀₁; is C₁₋₈ alkyl or C₁₋₃ alkylene substituted with a benzene ring, wherein the benzene ring is phenyl or phenyl substituted with 1-4 C₁₋₃ alkyl; optionally, R₀₂ is methyl or benzyl;
in formula (I-2), formula (I-3) and formula (I-4), L₂₃ is respectively and independently a linear or branched type; optionally, L₂₃ is respectively and independently linear C₈₋₂₀ hydrocarbylene; further optionally, L₂₃ is respectively and independently linear C₈₋₂₀ alkylene or linear C₈₋₂₀ alkenylene; still further optionally, L₂₃ is respectively and independently linear C₁₀₋₁₈ alkylene or linear C₁₀₋₁₈ alkenylene; still further optionally, L₂₃ is respectively and independently linear C₁₂₋₁₈ alkylene or linear C₁₂₋₁₈ alkenylene.

In some embodiments, the surfactant includes one or more of the following compounds, and may be any one or any suitable combination of the following compounds:

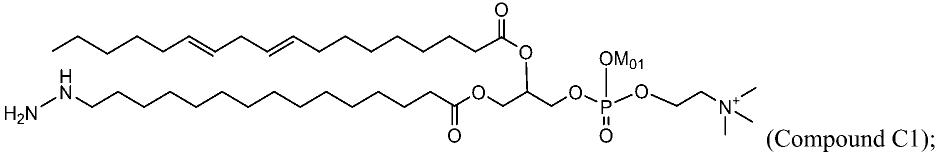

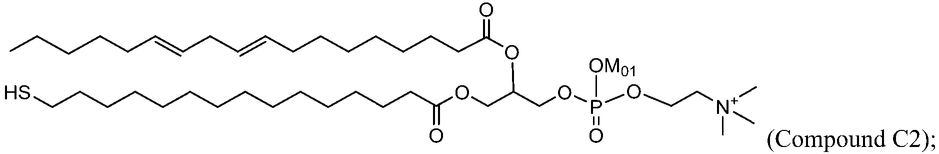

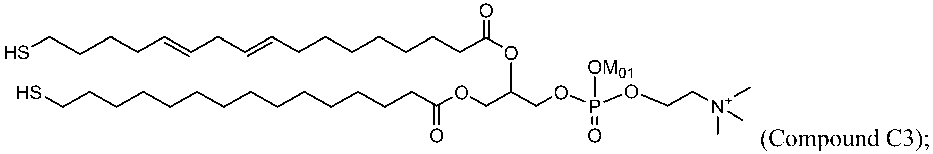

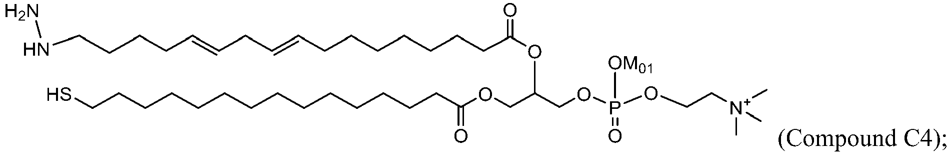

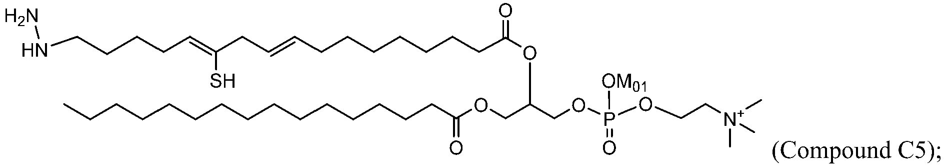

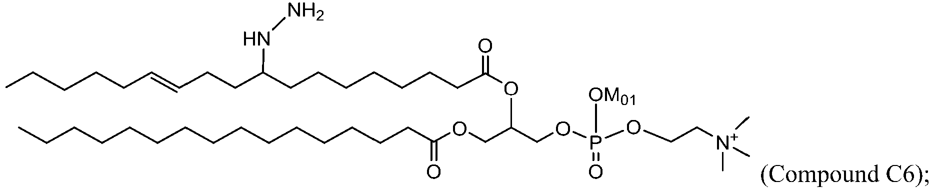

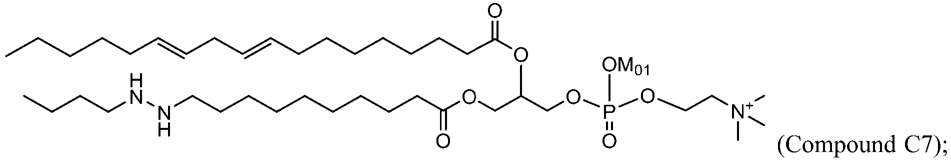

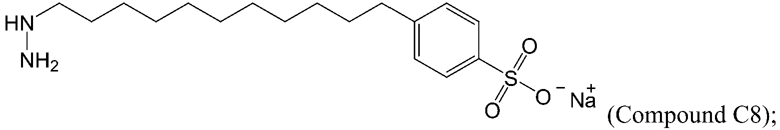

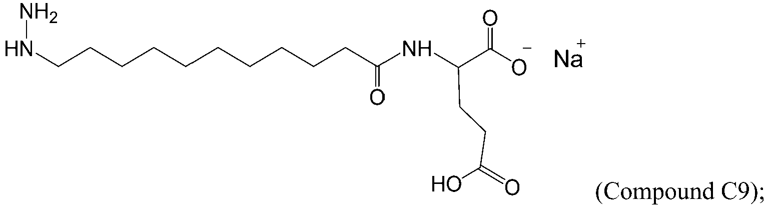

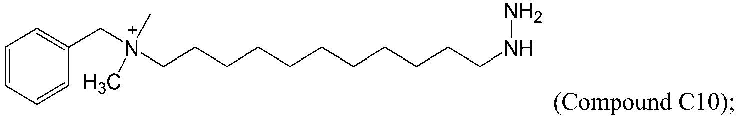

In the above compounds, the definition of M₀₁ is consistent with the above. In some embodiments, M₀₁ is absent, taking the compound C1 as an example, in this case it corresponds to

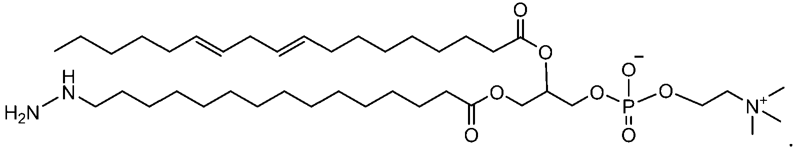

In some embodiments, the surfactant includes one or more of the following compounds, and may be any one or any suitable combination of the following compounds: the compound C1, the compound C2, the compound C3, the compound C4, the compound C5, the compound C6, the compound C8 and the compound C9.

When any of the above compounds contain chiral atoms, it may be in any suitable stereoisomeric form. For example, 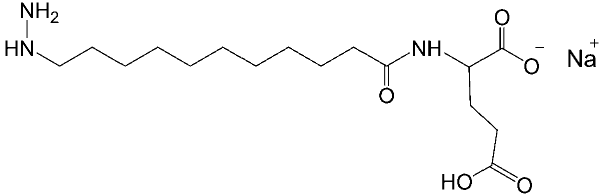 may be 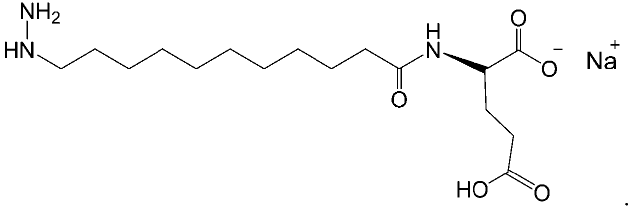 For another example, the following stereoisomer structures:

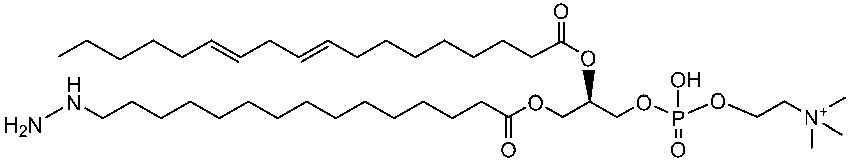

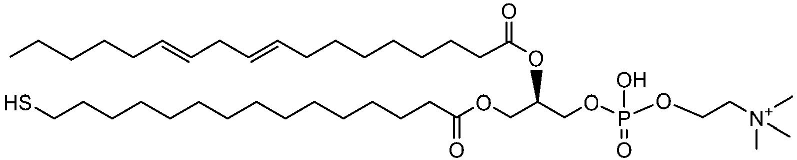

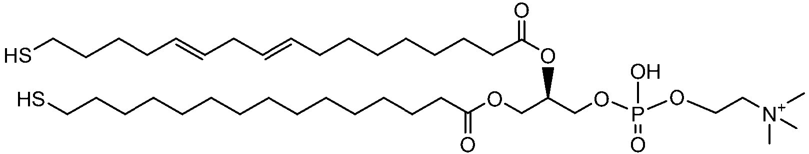

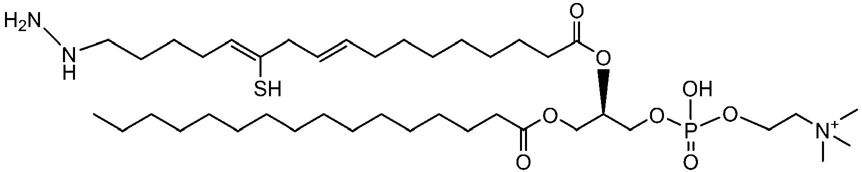

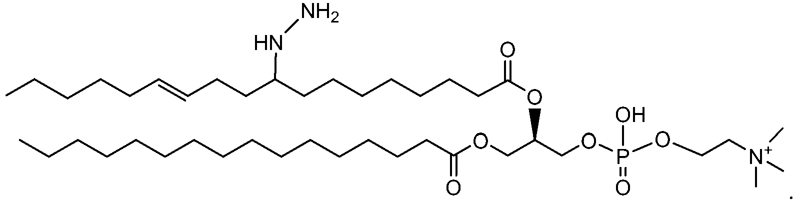

In some embodiments, the molar percentage of the surfactant relative to the divalent metal ion in the perovskite precursor material is in a range from 0.001 mol% to 5 mol%; optionally, the molar percentage of the surfactant relative to the divalent metal ion in the perovskite precursor material is in a range from 0.001% to 2.5%; further optionally, the molar percentage of the surfactant relative to the divalent metal ion in the perovskite precursor material is in a range from 0.01% to 1%; still further optionally, the molar percentage of the surfactant relative to the divalent metal ion in the perovskite precursor material is in a range from 0.01% to 0.5%. The molar percentage of the surfactant relative to the divalent metal ion in the perovskite precursor material may also be selected from any one of the following molar percentages: 0.001 mol%, 0.005 mol%, 0.01 mol%, 0.02 mol%, 0.04 mol%, 0.05 mol%, 0.06 mol%, 0.08 mol%, 0.1 mol%, 0.12 mol%, 0.14 mol%, 0.15 mol%, 0.16 mol%, 0.18 mol%, 0.2 mol%, 0.22 mol%, 0.24 mol%, 0.25 mol%, 0.26 mol%, 0.28 mol%, 0.3 mol%, 0.35 mol%, 0.4 mol%, 0.45 mol%, 0.5 mol%, 0.55 mol%, 0.6 mol%, 0.65 mol%, 0.7 mol%, 0.75 mol%, 0.8 mol%, 0.9 mol%, 1 mol%, 1.2 mol%, 1.4 mol%, 1.5 mol%, 1.6 mol%, 1.8 mol%, 2 mol%, 2.5 mol%, 3 mol%, 3.5 mol%, 4 mol%, 4.5 mol%. 5 mol% and the like, and may further be selected from an interval composed of any two molar percentages above, such as 0.01 mol%-2.5 mol%, 0.01 mol%-2 mol%, and 0.01 mol%-1 mol%.

The amount of the first functional group can be adjusted by adjusting the content of the surfactant, so as to better realize the synergistic effect of the surfactant and the perovskite precursor material while taking into account improving the wettability of the perovskite precursor solution and reducing the vacancy defects in the perovskite, thereby better improving the uniformity of the perovskite film, which is also more beneficial to improving the energy conversion efficiency of the battery.

In some embodiments, the solvent includes a first solvent and a second solvent, and a boiling point of the first solvent is lower than a boiling point of the second solvent. As non-limiting examples, the first solvent may include, but is not limited to one or more of N,N-dimethylformamide, 2-methoxyethanol and acetonitrile; and the second solvent may include, but is not limited to one or more of N-methylpyrrolidone, diphenyl sulfoxide, and dimethylpropyleneurea.

In the present application, a boiling point of the solvent may be tested using known methods and instruments. Unless otherwise specified, the boiling points of the first solvent and the second solvent refer to boiling points tested at a room temperature and a normal pressure. Unless otherwise specified, the "room temperature" here is such as 20-35°C, further such as 20-30°C, further such as 25°C. Unless otherwise specified, the "normal pressure" here may refer to one standard atmospheric pressure.

In some embodiments, a volume ratio of the first solvent to the second solvent is in a range from 3 to 10; optionally, the volume ratio of the first solvent to the second solvent is in a range from 3 to 5. The volume ratio of the first solvent to the second solvent may also be any of the following ratios: 3, 3.5, 4, 4.5, 5, 5.5, 6, 6.5, 7, 7.5, 8, 8.5, 9, 9.5, 10, etc., and may also be selected from an interval composed of any two ratios above.

Solvents with different boiling points can be used in combination to adjust a solvent evaporation rate and thus adjust and control the morphology of the perovskite thin film when preparing the perovskite thin film, thereby better improving the uniformity of the thin film and being more conducive to improving the energy conversion efficiency of the perovskite cell.

In some embodiments, the perovskite precursor material contains a perovskite-type metal halide; a chemical formula of the perovskite-type metal halide is ABX₃; where A is a monovalent cation, B is a divalent cation, and X is a monovalent anion.

In some embodiments, A includes one or more of Cs⁺, K⁺, Rb⁺, Li⁺, organic amine cations, etc. The organic amine cation may include one or more of a monovalent amine cation and a monovalent amidino cation.

Non-limiting examples of the monovalent amine cation are (NR₂₁R₂₂R₂₃R₂₄)⁺, (R₂₁R₂₂N=CR₂₃R₂₄)⁺, (R_{21R22}N-C(R₂₅)=NR₂₃R₂₄)⁺ or (R₂₁R₂₂N-C(NR₂₅R₂₆)=R₂₃R₂₄)⁺, wherein R₂₁, R₂₂, R₂₃, R₂₄, R₂₅ and R₂₆ are each independently selected from H, C₁₋₂₀ alkyl, aryl, substituted C₁₋₂₀ alkyl or substituted aryl; wherein the "C₁₋₂₀ alkyl" in the C₁₋₂₀alkyl and the substituted C₁₋₂₀ alkyl is each independently and optionally C₁₋₁₅ alkyl, further optionally C₁₋₁₀alkyl, still further optionally C₁₋₈ alkyl, still further optionally C₁₋₆alkyl, still further optionally C₁₋₄ alkyl, still further optionally C₁₋₃alkyl, and still further optionally methyl. The "aryl" in the aryl and the substituted aryl is each independently and optionally C₆₋₂₀ aryl, further optionally C₆₋₁₂ aryl, still further optionally C₆₋₁₀ aryl, still further optionally phenyl or naphthyl, still further optionally phenyl. The substituents in the substituted C₁₋₂₀ alkyl and the substituted aryl are each independently C₁₋₁₀ hydrocarbyl, further optionally C₁₋₆ alkyl or C₆₋₁₀ aryl, still further optionally methyl or phenyl.

Non-limiting examples of the monovalent amine cation further include CH₃NH₃⁺ (methylamine, MA⁺), ammonium (NH₄⁺). Non-limiting examples of the monovalent amidino cation are NH₂CH=NH₂⁺ (formamidine, which may be denoted as FA⁺).

In the present application, unless otherwise specified, B is a divalent metal ion.

In some embodiments, B includes one or more of Pb²⁺, Sn²⁺, Fe²⁺, Mn²⁺, Ni²⁺, Ge²⁺, Co²⁺ and Sb²⁺.

In some embodiments, B may include, but is not limited to, divalent metal ions of one or more of the following elements: lead, tin, zinc, titanium, antimony, bismuth, nickel, iron, cobalt, silver, copper, gallium, germanium, magnesium, calcium, indium, aluminum, manganese, chromium, molybdenum, europium, and the like.

In some embodiments, X includes one or more of I⁻, Br⁻, Cl⁻ and F⁻.

In some embodiments, X includes one or more of I⁻, Br⁻ and Cl⁻.

In some embodiments, X includes one or both of I⁻ and Br⁻. X may be I⁻, Br⁻or a combination thereof. In some embodiments, X is I⁻.

In a second aspect, the present application provides a perovskite thin film, prepared by coating and annealing the perovskite precursor solution described in the first aspect of the present application or at least containing a non-solvent component in the perovskite precursor solution described in the first aspect of the present application.

The perovskite thin film prepared using the perovskite precursor solution described in the first aspect of the present application has good contact with a substrate, the thin film is uniform and has few defects, and the corresponding perovskite cell has high energy conversion efficiency.

In some embodiments, the perovskite thin film is prepared by a method including the following steps: applying the perovskite precursor solution described in the first aspect of the present application to a preset position, and performing annealing treatment to prepare the perovskite thin film.

The coating may be performed by a slot coating method.

The annealing treatment temperature may be in a range from 100°C to 180°C, for example, 100°C, 110°C, 120°C, 130°C, 140°C, 150°C, 160°C, 180°C, etc., and may also be selected from a temperature interval composed of any two of the above temperatures, such as 100-150°C.

The uniformity of the perovskite thin film, especially the problem of the uniformity of the large-area perovskite thin film, is one of the important factors restricting the industrialization of perovskite solar cells. Currently, the mainstream method for preparing the large-area perovskite thin films is the coating method, which requires the perovskite precursor solution to have low surface tension and good wettability. For this reason, the surfactant is generally added to the perovskite precursor solution. However, if the added surfactant is inappropriate, it may cause the surfactants to form defects in perovskite crystals, resulting in deterioration of the performance of the perovskite solar cells.

In some embodiments, an area of the perovskite thin film is greater than or equal to 1 cm², further, for example, 1 cm², 1.5 cm², 2 cm², 3 cm², 4 cm², etc., or greater than or equal to any of the aforementioned areas, or selected from an interval composed of any two of the aforementioned areas.

In some embodiments, the area of the perovskite thin film is greater than or equal to 4 cm².

It is difficult to obtain a relatively uniform perovskite thin film for a large-area perovskite thin film (e.g ≥1 cm²). Compared with a perovskite precursor solution using a traditional surfactant, the perovskite precursor solution containing the surfactant having the aforementioned first functional group provided in the present application has significant advantages in improving the uniformity of the large-area perovskite thin film.

In a third aspect, the present application provides a perovskite cell including the perovskite thin film described in the second aspect of the present application.

In some embodiments, the present application provides a perovskite cell, including an electron transport layer, a hole transport layer, and the perovskite thin film described in the second aspect of the present application, wherein the perovskite thin film is disposed between the electron transport layer and the hole transport layer.

In some embodiments, the present application provides a perovskite cell, including a positive electrode, a negative electrode, and the perovskite thin film described in the second aspect of the present application, wherein the perovskite thin film is disposed between the positive electrode and the negative electrode.

In some embodiments, the present application provides a perovskite cell, including a positive electrode, an electron transport layer, the perovskite thin film described in the second aspect of the present application, a hole transport layer and a negative electrode which are arranged in sequence. Further, the perovskite cell may be any one of a trans p-i-n cell and a formal n-i-p cell.

The perovskite thin film described in the second aspect of the present application may also be referred to as a perovskite layer, which is also a light-absorbing layer in the perovskite cell.

When the perovskite cell is working, after the light-absorbing layer is exposed to light, internal electrons gain energy and break free from the light-absorbing layer to form negatively-charged electron carriers, and at the same time form positively-charged hole carriers, thereby obtaining electron-hole pairs. Free electrons and free holes are transmitted in opposite directions through the corresponding transport layers, causing the electrons and the holes to flow, forming external current and realizing conversion of light energy into electrical energy. Further, after the perovskite layer absorbs photons, it is stimulated to generate electron-hole pairs, and the electron-hole pairs further dissociate to form free carriers with opposite charges. The free electrons are transmitted to the positive electrode through the electron transport layer, and the free holes are transmitted to the negative electrode through the hole transport layer. The two free carriers are respectively collected by the corresponding electrodes, further forming photocurrent in the circuit of the perovskite cell.

The electron transport layer can extract and transport the electron carriers and block the passage of the free holes.

The hole transport layer can extract and transport the hole carriers and block the passage of the free electrons.

It can be understood that the perovskite cell further includes two electrodes. One of the two electrodes serves as a positive electrode and can collect the electron carriers transmitted via the electron transport layer, and the other serves as a negative electrode and can collect the hole carriers transmitted via the hole transport layer.

In some embodiments, the electron transport layer material may include, but is not limited to one or more of the following materials and derivatives thereof: an imide compound, a quinone compound, fullerene and derivatives thereof, methoxytriphenylamine fluoroformamidine (OMeTPA-FA), calcium titanate (CaTiO₃), lithium fluoride (LiF), calcium fluoride (CaF₂), poly(3,4-ethylenedioxythiophene):polystyrene sulfonic acid (PEDOT: PSS), poly-3-hexylthiophene (P3HT), triptycene-cored triphenlamine (H101), 3,4-ethylenedioxythiophenemethoxytriphenylamine (EDOT OMeTPA), (4-aniline)carbazole-spirobifluorene (CzPAF-SBF), polythiophene, a metal oxide, silicon oxide (SiO₂), strontium titanate (SrTiO₃), cuprous thiocyanate (CuSCN), etc; wherein the metal elements may include one or more of Mg, Ni, Cd, Zn, In, Pb, Mo, W, Sb, Bi, Cu, Hg, Ti, Ag, Mn, Fe, V, Sn, Zr, Sr, Ga and Cr.

In some embodiments, the hole transport layer may include, but is not limited to, one or more of the following materials and derivatives thereof: 2,2',7,7'-tetrakis[N,N-di(4-methoxyphenyl)amino]-9,9'-spirobifluorene (Spiro-OMeTAD), polytriarylamine (PTAA), a nickel oxide (NiOₓ), poly-3,4-ethylenedioxythiophene:polystyrene sulfonate (PEDOT:PSS), WO₃and other materials that can transport the holes and block the electrons.

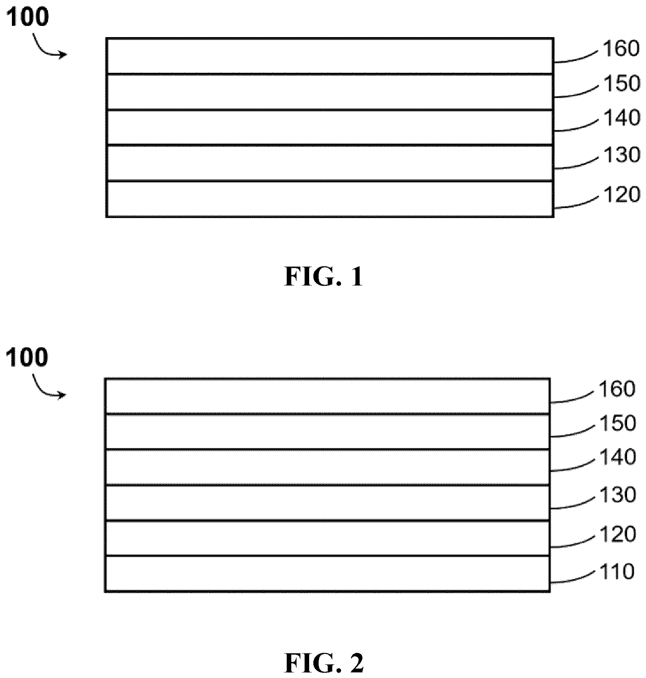

In some examples, the perovskite cell 100 includes a strcture shown in FIG. 1, and includes a first electrode 120, a first transport layer 130, a perovskite layer 140, a second transport layer 150 and a second electrode 160 which are disposed in sequence. Further, the structural layers shown in the figure are stacked in sequence.

In some examples, the perovskite cell 100 includes a strcture shown in FIG. 2, and includes a substrate layer 110, a first electrode 120, a first transport layer 130, a perovskite layer 140, a second transport layer 150 and a second electrode 160 which are disposed in sequence. Further, the structural layers shown in the figure are stacked in sequence.

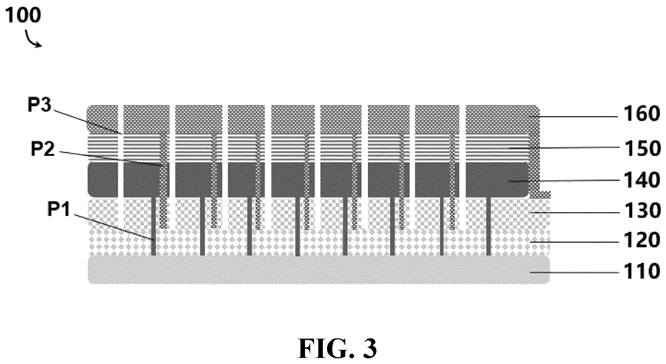

In some examples, the perovskite cell includes a strcture shown in FIG. 3 (which is a vertical cross-sectional structure diagram of the device), and includes a substrate layer 110, a first electrode 120, a first transport layer 130, a perovskite layer 140, a second transport layer 150 and a second electrode 160 which are stacked in sequence. Wherein P1, P2 and P3 are etching regions disposed across layers, and are used to divide the film layer prepared in a large area into different components to make it a series cell structure; and, P1, P2 and P3 are respectively used to connect the structural layers disposed apart, so that the structural layer between the first electrode and the second electrode forms a loop, and the perovskite cell is formed into a perovskite cell assembly. P1, P2 and P3 may be each independently a linear etching region, also called an etching line. P1, P2 and P3 may be each independently a laser etching region. The number of P1, P2 and P3 may be each independently one or more. In FIG. 3, P1 penetrates the first transport layer and a bottom of the first electrode from the surface of the first transport layer to be connected to the substrate layer, so that the left and right sides of the divided P1 are not connected to each other (to achieve insulation), and a material in the P1 etching region is consistent with that in the perovskite layer; P2 penetrates the second transport layer, the perovskite layer, and the first transport layer from the surface of the second transport layer to be connected to the surface of the first electrode, and a material in the P2 etching region is consistent with that of the second electrode; P3 penetrates the second electrode, the second transport layer, the perovskite layer, the first transport layer from the surface of the second electrode to the surface of the first electrode, and no material is filled in the P3 etching region.

In some embodiments, a width of P1 is in a range from 10 µm to 50 µm, such as 30 µm.

In some embodiments, a width of P2 is in a range from 10 µm to 200 µm, such as 150 µm. Further, an interval between P2 and P1 may be in a range from 20 µm to 80 µm, such as 20 µm.

In some embodiments, a width of P3 is in a range from 10 µm to 50 µm, such as 15 µm. Further, an interval between P3 and P2 may be in a range from 20 µm to 40 µm, such as 20 µm.

In some embodiments, P1 in the perovskite cell may penetrate from the surface of the first transport layer to the bottom of the first electrode, and the filling material in the P1 is consistent with that in the perovskite layer (as shown in FIG. 3). In other pther embodiments, P1 in the perovskite cell may further penetrate from the surface of the first electrode to the bottom, and the filling material in the P1 is consistent with that in the first transport layer.

In some embodiments, one of the "first transport layer" and the "second transport layer" is an electron transport layer, and the other is a hole transport layer. In some embodiments, the first transport layer is the electron transport layer. In some embodiments, the first transport layer is the hole transport layer.

In some examples, one of the "first electrode" and the "second electrode" is a transparent electrode, and used for light incidence. In some embodiments, the first electrode is the transparent electrode.

In some embodiments, the material of the transparent electrode may be exemplified by, but not limited to, one or more of the following materials: fluorine-doped tin oxide (FTO), tin-doped indium oxide (ITO), aluminum-doped zinc oxide (AZO), boron-doped zinc oxide (BZO), indium zinc oxide (IZO), tungsten-doped indium oxide (IWO), etc.

In some embodiments, the second electrode contains a conductive material, and further, the conductive material may be an organic conductive material, an inorganic conductive material, or a combination thereof. Non-limiting examples of the inorganic conductive material are a metal conductive material. Further, the metal conductive material may include any one of gold (Au), silver (Ag), copper (Cu), aluminum (Al), nickel (Ni), chromium (Cr), bismuth (Bi), platinum (Pt), magnesium (Mg), etc., or any suitable mixture of the aforementioned elements. The conductive material may include a conductive oxide. Further, the conductive material may be a conductive oxide; non-limiting examples of the conductive oxide may include one or more of FTO, ITO, IWO, AZO, etc..

In some embodiments, the perovskite cell is any one of a trans p-i-n cell and a formal n-i-p cell.

The perovskite cell provided by the present application may include both formal and trans forms.

For the formal form, the perovskite cell includes a transparent electrode, as well as an electron transport layer, a perovskite layer, a hole transport layer and a second electrode layer which are stacked in sequence on the transparent electrode.

For the trans form, the perovskite cell includes a transparent electrode, as well as a hole transport layer, a perovskite layer, an electron transport layer and a second electrode layer which are stacked in sequence on the transparent electrode. The transparent electrode is used for light incidence.

In some embodiments, the perovskite cell includes the following structures disposed in sequence: a substrate layer (which may be a glass substrate or a flexible substrate), a first electrode, a hole transport layer, a perovskite layer, an electron transport layer, and a second electrode. The flexible substrate may include one or more materials of polyethylene terephthalate, polyimide, polyethylene, polypropylene, polystyrene, polyethylene naphthalate, etc. Optionally, the first electrode is a transparent electrode used for light incidence.

In some embodiments, the perovskite cell includes the following structures disposed in sequence: a substrate layer (a glass substrate or a flexible substrate), a first electrode, an electron transport layer, a perovskite layer, a hole transport layer, and a second electrode. Optionally, the first electrode is a transparent electrode used for light incidence. The definition of flexible substrate can be found above.

The substrate layer involved in the embodiment or example of the present application may be, but is not limited to, the glass substrate or the flexible substrate.

In some embodiments, the substrate layer is a flexible substrate layer. Furthermore, the material of the substrate layer may be, for example (but not limited to), an organic polymer material. Furthermore, it may be a mixture of one or more of the following materials in different proportions: including but not limited to polyvinyl alcohol (PVA), polyester (PET), polyimide (PI), polyethylene naphthalate (PEN), polydimethylsiloxane (PDMS), etc.

In some embodiments, the substrate layer 110 in the structure shown in FIG. 3 is a light-incident glass substrate.

The specification of the perovskite cell is not particularly limited and may be, but not limited to, 300 mm×300 mm.

It can be understood that the structure of the perovskite cell involved in the present application may not be limited to the structural layers listed above. Other functional layers, such as a buffer layer, may also be introduced as required. In some embodiments, the perovskite cell may be provided with a buffer layer with a suitable energy level, which may play one or more of the roles of reducing energy level barriers, promoting energy level matching, and improving carrier extraction efficiency, while also passivating interface defect states, protecting a light absorption layer, inhibiting water molecules and oxygen from oxidative decomposition of the cell, improving photoelectric conversion efficiency, and improving the stability of the perovskite cell. Depending on the position of the buffer layer, the types of the buffer layer may include four types: a buffer layer between the hole transport layer and an anode, a buffer layer between the electron transport layer and a cathode, a buffer layer between the hole transport layer and an absorption layer, and a buffer layer between the electron transport layer and the absorption layer. Materials that can be used for the buffer layer in the perovskite cell may include, but are not limited to: Cu₂O, NiO, AZO, TiO₂, etc.

In a fourth aspect, the present application provides an electrical apparatus, including the perovskite cell described in the third aspect of the present application.

The structure of the perovskite cell may be, but is not limited to, a single structure, a stacked structure, or other structures.

In some of the embodiments, the perovskite cell above may be used as a power generation device for the electrical apparatus. Types of power generation apparatuses may include, but are not limited to, integrated power generation. Positions of the power generation apparatuses may include, but are not limited to the roof, back panel, etc. of the car.

Further, the above electrical apparatus may include mobile equipment, such as a mobile phone and laptop computer, an electric vehicle, an electric train, a ship, a satellite, a power generation system, etc., but are not limited thereto.

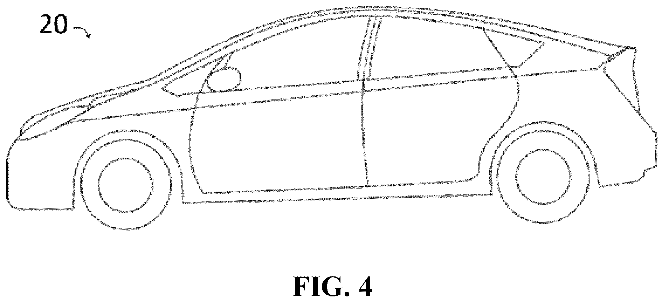

FIG. 4 is an electrical apparatus as an example. The electrical apparatus 20 is a car, and may further be an all-electric vehicle, a hybrid electric vehicle, a plug-in hybrid electric vehicle, or the like.

As another example, the electrical apparatus may be a mobile phone, a tablet personal computer, a laptop, a calculator, etc.

As another example, the electrical apparatus may be wearable equipment such as a watch. Some examples of the present application are described below. The embodiments described below are illustrative, are merely used to explain the present application, and should not be construed as limiting the present application. The embodiments in which techniques or conditions are not specified are implemented based on the description above, or based on techniques or conditions described in literatures of the art, or based on the product manuals. Where no manufacturers are specified, the employed reagents or instruments are all commercially available conventional products, or can be synthesized by the commercially available products in a conventional way.

In the following examples, the room temperature refers to 20°C to 30°C, and further, may be 25°C.

In the following examples, unless otherwise specified, the following raw materials may be obtained commercially or may be prepared by simple chemical modification of commercially available raw materials. The intermediate and final products synthesized in all examples, including the surfactant of the present application, can be structurally identified by one or more of the following detection methods, including but not limited to: Fourier transform infrared (FT-IR) spectroscopy, ultraviolet spectroscopy, hydrogen nuclear magnetic resonance (¹H NMR), gel permeation chromatography (GPC), high performance liquid chromatography (HPLC), mass spectrometry, etc. Sample preparation methods and test methods of these test methods are known to those skilled in the art, and test parameters can be appropriately adjusted according to the specific structure and material properties of the compound. For those skilled in the art, in a case where the structural formula of a target compound is given, whether the compound with the target structure has been synthesized can be judged by combining the test results of the aforementioned test methods. Taking ¹H NMR as an example, structural identification can be performed according to parameters such as a peak position, a peak shape, and an integrated area ratio in the hydrogen nuclear magnetic spectrum to confirm whether a specific group disappears or appears.

In the following examples, unless otherwise specified, NBS is N-bromosuccinimide, DMF is N,N-dimethylformamide, and DMSO is dimethyl sulfoxide.

### Preparation Example: preparation of surfactant

¹H NMR tests were performed using a Bruker AVANCE NEO nuclear magnetic resonance (NMR) spectrometer with tetramethylsilane (TMS) as the internal standard, a scan power of 400 MHz, and deuterated dimethylformamide (dDMF) as the solvent.

High performance liquid chromatography (HPLC) test: Thermo Fisher Ultimate 3000 standard; solvent: acetonitrile.

Mass spectrometry test: Shimadzu gas chromatograph/mass spectrometer GCMS-QP2010; solvent: acetone.

Lecithin uses CAS: 8002-43-5. In the following Preparation Examples 1-8, the lecithin raw materials involved have the same source.

### Preparation Example 1. Preparation of hydrazino-modified lecithin (Sul)

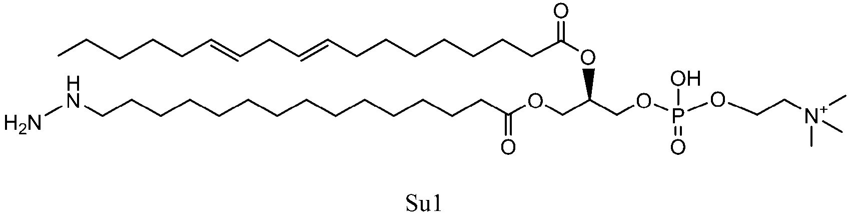

### Step 1: synthesis of brominated lecithin

a clean and dry round-bottom flask was taken, 5.4 g of NBS, 0.03 mol of AuCl₃, 60 mL of dichloroethane, 0.06 mol of lecithin (CAS: 8002-43-5) and a magneton ware added, and reacted at 80°C for 11 hours, and a reaction process was monitored by high performance liquid chromatography (HPLC); and if there is still raw material remaining after 11 hours, a reaction time was extended appropriately, when the reaction is complete, insoluble matter was removed by filtering, a mother liquor was concentrated by vacuum distillation, and the brominated lecithin was obtained by column chromatography (a mobile phase was petroleum ether/ethyl acetate with a volume ratio of 20:1).

### Step 2: synthesis of hydrazino-modified lecithin

Another clean and dry round-bottom flask was taken, 0.07 mol of K₂CO₃, 0.07 mol of NaI and 0.07 mol of hydrazine hydrate were added in sequence to 0.007 mol of brominated lecithin, and then 100 mL of DMF and magneton were added, and stirred to obtain a suspension. A mixture was heated to 80°C and reacted for 24 hours, filtered in turn, and concentrated to remove the solvent. The mixture was extracted repeatedly with a saturated salt solution and dichloromethane, an oil phases was mixed and concentrated, and a product was purified by column chromatography (the mobile phase was ethyl acetate/n-hexane = 1:10 of volume ratio). The product was dried in vacuum at room temperature for 16 hours to obtain the hydrazino-modified lecithin. After compound testing and structural analysis, it was confirmed that the compound with the structure shown in Su1 was obtained, and was recorded as compound Su1.

### Preparation Example 2. Preparation of thiol-modified lecithin (Su2)

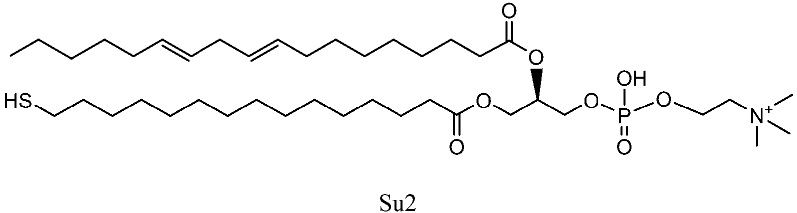

The thiol-modified lecithin was synthesized by a method substantially the same as that in Preparation Example 1, except that in step 2:
0.05 mol of brominated lecithin and 0.052 mol of thiourea were dissolved in 50 mL of 95% ethanol, and a reaction mixture was refluxed and stirred for 3 hours. Then, 30 mL of 2.5 mol/L sodium hydroxide solution was added to the reaction mixture, and reflux was continued for 2 hours. An aqueous layer was separated and acidified (pH 1) by the addition of dilute hydrochloric acid. An acidic solution was then extracted twice with petroleum ether. Organic phases were combined and washed with brine. After the organic phase was dried over sodium sulfate, the petroleum ether solvent was removed by distillation, the product was separated by column chromatography, and was dried in vacuum at room temperature for 8 hours to obtain the thiol-modified lecithin. After compound testing and structural analysis, it was confirmed that the compound with the structure shown in Su2 was obtained, and was recorded as compound Su2.

Wherein 95% ethanol refers to a mixed liquor of ethanol and water in a volume ratio of 95%:5%.

### Preparation Example 3. Preparation of hydrazino-modified sodium dodecylbenzene sulfonate (Su3)

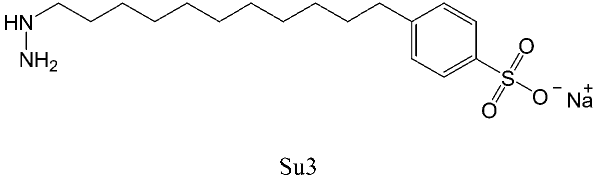

The hydrazino-modified sodium dodecylbenzene sulfonate was synthesized by a method substantially the same as that in Preparation Example 1, except that in step 1, lecithin was replaced with sodium dodecylbenzene sulfonate. After compound testing and structural analysis, it was confirmed that the compound with the structure shown in Su3 was obtained and was recorded as compound Su3.

### Preparation Example 4. Preparation of hydrazino-modified sodium lauroyl glutamate (Su4)

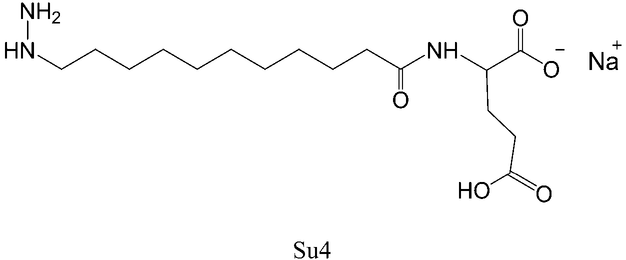

The hydrazino-modified sodium lauroyl glutamate was synthesized by a method substantially the same as that in Preparation Example 1, except that in step 1, lecithin was replaced with sodium lauroyl glutamate. After compound testing and structural analysis, it was confirmed that the compound with the structure shown in Su4 was obtained and was recorded as compound Su4.

### Preparation Example 5. Preparation of dithiol-modified lecithin (Su5)

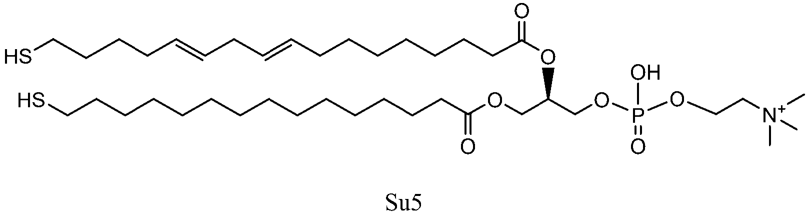

The dithiol-modified lecithin was synthesized by a method substantially the same as that in Preparation Example 2, except that the feeding amount of NBS in step 1 was doubled (changed to 10.8 g), and meanwhile, the usage amount of brominated lecithin in step 2 was reduced to half of the original amount, i.e., 0.025 mol. After compound testing and structural analysis, it was confirmed that the compound with the structure shown in Su5 was obtained and was recorded as compound Su5.

### Preparation Example 6. Preparation of hydrazino- and thiol-modified lecithin (Su6)

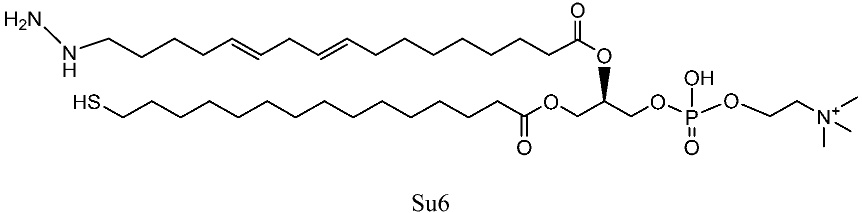

The hydrazino- and thiol-modified lecithin was synthesized by a method substantially the same as that in Preparation Example 1, except that step 3 was added after step 2 in Preparation Example 1:
0.05 mol of hydrazino-modified lecithin and 0.052 mol of thiourea were dissolved in 50 mL of 95% ethanol, and a reaction mixture was refluxed and stirred for 3 hours. Then, 30 mL of 2.5 mol/L sodium hydroxide solution was added to the reaction mixture, and reflux was continued for 2 hours. An aqueous layer was separated and acidified (pH 1) by the addition of dilute hydrochloric acid. An acidic solution was then extracted twice with petroleum ether. Organic phases were combined and washed with brine. After the organic phase was dried over sodium sulfate, the petroleum ether solvent was removed by distillation, the product was separated by column chromatography, and was dried in vacuum at room temperature for 8 hours to obtain the hydrazino- and thiol-modified lecithin. After compound testing and structural analysis, it was confirmed that the compound with the structure shown in Su6 was obtained, and was recorded as compound Su6.

### Preparation Example 7. Preparation of hydrazino- and thiol-modified lecithin (Su7)

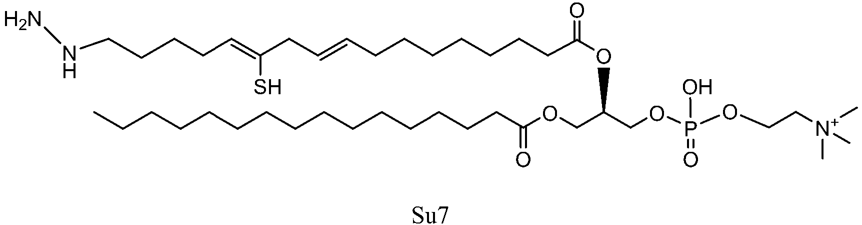

### Step 1: synthesis of brominated lecithin

a clean and dry round-bottom flask was taken, 5.4 g of NBS, 0.03 mol of AuCl₃, 60 mL of dichloroethane, 0.06 mol of lecithin (CAS: 8002-43-5) and a magneton ware added, and reacted at 80°C for 11 hours, and a reaction process was monitored by liquid chromatography; and if there was still raw material remaining after 11 hours, a reaction time was extended appropriately, when the reaction is complete, insoluble matter was removed by filtering, a mother liquor was concentrated by vacuum distillation, and the brominated lecithin was obtained by column chromatography (a mobile phase was petroleum ether/ethyl acetate with a volume ratio of 20:1).

### Step 2: synthesis of hydrazino-modified lecithin

Another clean and dry round-bottom flask was taken, 0.07 mol of K₂CO₃, 0.07 mol of NaI and 0.07 mol of hydrazine hydrate were added in sequence to 0.007 mol of brominated lecithin (prepared in step 1), and then 100 mL of DMF and magneton were added, and stirred to obtain a suspension. A mixture was heated to 80°C and reacted for 24 hours, filtered sequentially, and concentrated to remove the solvent. The mixture was extracted repeatedly with a saturated salt solution and dichloromethane, an oil phases was mixed and concentrated, and a product was purified by column chromatography (the mobile phase was ethyl acetate/n-hexane = 1:10 of volume ratio). The product was dried in vacuum at room temperature for 16 hours to obtain the hydrazino-modified lecithin IM7b.

Step 3: synthesis of BMPO ligand: 5 mmol of 2,6-dimethylaniline was accurately weighed, and dissolved in 10 mL of tetrahydrofuran (THF) solution, 10.5 mmol of triethylamine was added in turn and stirred, 10 mmol of oxalyl chloride was added slowly under ice-water bath, and a mixture was stirred at a room temperature for 3 hours, and concentrated in vacuum to remove residual solvent. Water was added to dissolve and filtered to remove Et₃N·HCl (triethylamine hydrochloride). A filtrate was washed with water and petroleum ether and dried in vacuum to obtain the BMPO ligand.

Step 4: a mixture of the product IM7b (4mmol) in step 2, LiBr (40 mmol),CF₃CO₂H (12 mmol) and Cu complex (8 mol% CuI, 8 mol% BMPO, 16 mol% hexadecyltrimethylammonium bromide (CTAB)) in CH₃NO₂ (10 mL) was charged into a reaction tube, and the mixture was heated at 60°C for 20 hours under an oxygen atmosphere. The mixture was filtered in turn and concentrated to remove the solvent. The mixture was extracted repeatedly with a saturated salt solution and dichloromethane, an oil phases was mixed and concentrated, and a product IM7c was purified by column chromatography (the mobile phase was ethyl acetate/n-hexane = 1:10 of volume ratio).

Step 5: 5 mmol of the product IM7c in step 3 and 0.052 mol of thiourea were dissolved in 50 mL of 95% ethanol, and a reaction mixture was refluxed and stirred for 3 hours. Then, 30 mL of 2.5 mol/L sodium hydroxide solution was added to the reaction mixture, and reflux was continued for 2 hours. An aqueous layer was separated and acidified (pH 1) by the addition of dilute hydrochloric acid. An acidic solution was then extracted twice with petroleum ether. Organic phases were combined and washed with brine. After the organic phase was dried over sodium sulfate, the petroleum ether solvent was removed by distillation, the product was separated by column chromatography, and was dried in vacuum at room temperature for 8 hours to obtain the hydrazino- and thiol-modified lecithin. After compound testing and structural analysis, it was confirmed that the compound with the structure shown in Su7 was obtained, and was recorded as compound Su7.

Wherein CF₃CO₂H is trifluoroacetic acid.

### Preparation Example 8. Preparation of hydrazino-modified lecithin (Su8)

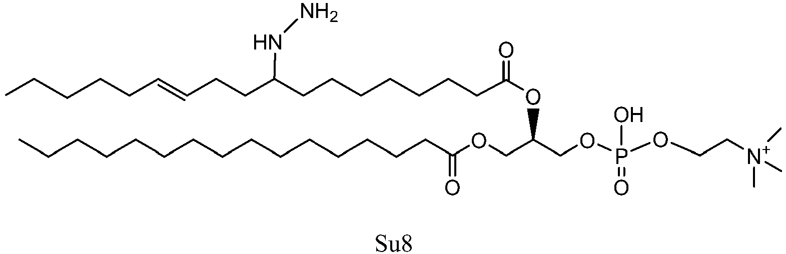

### Step 1 : preparation of 2-((((2S)-2-(((E)-9-bromooctadec-12-enoyl)oxy)-2-(palmitoyloxy)ethoxy)(hydroxy)phosphoryl)oxy)-N,N,N-trimethylethane-1-amine (preparation of brominated lecithin)

A clean and dry three-necked round-bottom flask was taken, a thermometer and a condenser tube were inserted, 5 mmol of lecithin (CAS: 8002-43-5) was added and cooled to -5°C, 10 mmol of hydrobromic acid was dropwise, and the temperature during the dropwise addition process was controlled to be 0-5°C. After the dropwise addition is completed, the reaction was continued for 1 hour, vacuum distillation was performed to obtain brominated lecithin P8a. The brominated lecithin was concentrated, and the product was purified by column chromatography (ethyl acetate/n-hexane = 1:20, volume ratio), and dryed in vacuum at 50°C for 12 hours. After structural testing and analysis, it was confirmed that the brominated compound P8a with the following structure was synthesized.

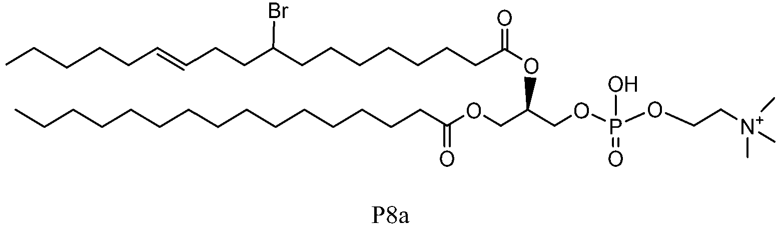

Step 2: synthesis of BMPO ligand: 5 mmol of 2,6-dimethylaniline was accurately weighed, and dissolved in 10 mL of tetrahydrofuran (THF) solution, 10.5 mmol of triethylamine was added in turn and stirred, 10 mmol of oxalyl chloride was added slowly under ice-water bath, and a mixture was stirred at a room temperature for 3 hours, and concentrated in vacuum to remove residual solvent. Water was added to dissolve and filtered to remove Et₃N·HCl (triethylamine hydrochloride). A filtrate was washed with water and petroleum ether and dried in vacuum to obtain the BMPO ligand.

Step 3: a clean and dry round-bottom flask was taken, 0.6 mmol of brominated compound P8a, 8mol% CuI, 8mol% BMPO, and 16mol% hexadecyltrimethylammonium bromide (CTAB) was added in sequence, and dissolved in 0.6 mL of water, 0.12 mmol of K₃PO₄ was added first, stirred with a magneton at 80°C for 15 minutes, then the remaining 0.6 mmol of K₃PO₄and 0.6 mmol of hydrazine hydrate of water were added, nitrogen passes through the mixture for 10-15 minutes, the oil bath temperature was heated to 80°C, sealing was performed for reaction for 8 hours, and the mixture was cooled to the room temperature, diluted with dichloromethane, filtered, and extracted with saturated salt solution; the organic layer was separated, 37% hydrochloric acid was added to acidify to pH 3.4, the precipitate was filtered, and washed with dichloromethane, the oil phase was mixed and concentrated, the product was obtained by purifying by column chromatography (ethyl acetate/n-hexane = 1:10), and the product was dried in vacuum at the room temperature for 16 hours. After compound testing and structural analysis, it was confirmed that the compound with the structure shown in Su8 was obtained.

### Example 1 Preparation of perovskite cell

A trans perovskite cell was taken as a non-limiting example. A nicking method shown in FIG. 1 was used. For nicking regions P1, P2 and P3, where a width of P1 is about 30 µm, a width of P2 is 150 µm, and a depth is etched to the FTO layer; an interval between P2 and P1 is 20 µm; and a width of P3 is 15 µm, a depth is etched to the FTO layer, and an interval between P3 and P2 is 20 µm.
1.1. A set of FTO conductive glass (as the first electrode) with a specification of 10cm×10cm was taken, and etched to form a P1 nick region, the P1 nick region was cleaned with a cleaning agent, and then ultrasonicated in deionized water, ethanol, and acetone for 10 minutes in succession; and after ultrasonication, the P1 nick region was blow-dried with N₂ for later use.
1.2. A piece of FTO conductive glass prepared in step 1.1 above was taken, and a nickel oxide layer (a hole transport layer as a first transport layer) with a thickness of 10 nm was prepared by magnetron sputtering.

### 1.3. Preparation of perovskite layer

Preparation of perovskite precursor solution: a solution of perovskite metal halide FAPbI₃ (1 mol/L) was prepared, the solvent was a mixed solvent of DMF and DMSO in a volume ratio of 4:1, and a surfactant was added according to the type and usage amount in Table 1 based on the molar percentage of the surfactant relative to Pb to obtain the perovskite precursor solution.

A layer of perovskite wet film was coated on the nickel oxide layer using the prepared perovskite precursor solution by a coating method, then the nickel oxide layer was transferred to vacuum equipment for evacuation for 60 seconds with a vacuum degree of 15 Pa, and the evacuated wafer was then placed on a hot plate at 120°C for annealing for 40 minutes to form a perovskite layer with a thickness of 500 nm. A complete perovskite layer was prepared and etched to form a P2 nick region.

### 1.4. Preparation of C60/BCP electron transport layer (second transport layer) and second electrode

After the perovskite is prepared, the wafer was placed in evaporation equipment, and 25nm C60, BCP (bathocuproine) with a thickness of 5 nm and 100nm Cu were evaporated in sequence; after the Cu electrode was formed, a P3 nick region was formed by etching; and a perovskite device was obtained.

**Examples 2-8** adopt substantially the same method as Example 1, except that the types and/or usage amounts of surfactants are different, see Table 1.

**Comparative Example 1** adopts substantially the same method as Example 1, except that lecithin is used as a surfactant.

**Comparative Example 2** adopts substantially the same method as Example 3, except that sodium dodecylbenzene sulfonate is used as a surfactant.

**Comparative Example 3** adopts substantially the same method as Example 4, except that sodium lauroyl glutamate is used as a surfactant.

**Comparative Example 4** adopts substantially the same method as Example 1, except that hydrazine hydrate with the molar weight equal to that of lecithin is further added.

**Comparative Example 5** adopts substantially the same method as Example 1, except that thioglycolic acid with the molar weight equal to that of lecithin is further added.

**Comparative Example 6** adopts substantially the same method as Example 1, except that no surfactant is added, and a DMF/DMSO solution of FAPbI₃is used as a precursor solution to prepare the perovskite thin film.

**Table 1.**

| Serial No. | Surfactant Type | Molar ratio concentration of surfactant relative to FAPbI₃ (mol%) | Molar ratio concentration of first functional group relative to FAPbI₃ (mol%) |
|---|---|---|---|
| Example 1 | Compound Su1 | 0.01 | 0.01 |
| Example 2 | Compound Su2 | 0.01 | 0.01 |
| Example 3 | Compound Su3 | 0.5 | 0.5 |
| Example 4 | Compound Su4 | 2.5 | 2.5 |
| Example 5 | Compound Su5 | 0.01 | 0.02 |
| Example 6 | Compound Su6 | 0.01 | 0.02 |
| Example 7 | Compound Su7 | 0.01 | 0.02 |
| Example 8 | Compound Su8 | 0.01 | 0.02 |
| Comparative Example 1 | Lecithin | 0.01 | 0 |
| Comparative Example 2 | Sodium dodecylbenzene sulfonate | 0.5 | 0 |
| Comparative Example 3 | Sodium lauroyl glutamate | 2.5 | 0 |
| Comparative Example 4 | Hydrazine hydrate + lecithin (equal molar weight) | 0.01 | 0.01 |
| Comparative Example 5 | Thioglycolic acid + lecithin (equal molar weight) | 0.01 | 0.01 |
| Comparative Example 6 | None | 0 | 0 |

### Test method:

### 1. Morphology of perovskite thin film

Test method: step profiler (Bruker DektakXT, resolution 10nm) thickness test and scanning electron microscope (Zeiss Sigma300; resolution 1nm).

The step profiler test can judge whether the film formation thickness is uniform, and the scanning electron microscope can observe whether the grain size is uniform. Among them, thickness deviation <5% is considered to be good uniformity, and grain size ±50nm is considered to be uniform.

### 2. Energy conversion efficiency and cell stability

The energy conversion efficiency of the cell was tested under standard simulated sunlight (AM 1.5G, 100 mW/cm²). The initial efficiency and the efficiency after 800 hours in dark state and RH<2% were tested respectively. The percentage of the efficiency value after 800 hours relative to the initial efficiency was recorded as the cell stability. The test results can be found in Table 2.

### Test results

According to the test results, it can be seen that the surfactant provided in the present application is used to prepare the perovskite precursor solution, and then prepare the perovskite thin film and the perovskite cell (see Examples 1-8), compared with Comparative Examples 1-6, the perovskite cells prepared in Examples 1-8 have significantly improved energy conversion efficiency and cell stability. In addition, the perovskite thin film with good uniformity and uniform grain size are obtained. This is because the surfactant provided in the present application significantly improves the uniformity of the perovskite thin film. Wherein no surfactant is added in Comparative Example 6, Comparative Examples 1-3 use common surfactants, and Comparative Examples 4-5 use a combination of common surfactants and functionalized small molecules with lone pairs.

**Table 2.**

| Serial No. | Cell efficiency (Initial) | Cell efficiency (After 800 hours) | Cell stability (%) |
|---|---|---|---|
| Example 1 | 19.26 | 19.01 | 98.70 |
| Example 2 | 19.10 | 18.98 | 99.37 |
| Example 3 | 18.84 | 18.53 | 98.35 |
| Example 4 | 18.97 | 18.79 | 99.05 |
| Example 5 | 18.57 | 18.49 | 99.57 |
| Example 6 | 19.03 | 18.87 | 99.16 |
| Example 7 | 18.42 | 17.86 | 96.96 |
| Example 8 | 18.35 | 17.99 | 98.04 |
| Comparative Example 1 | 18.32 | 17.58 | 95.96 |
| Comparative Example 2 | 17.54 | 16.90 | 96.35 |
| Comparative Example 3 | 17.98 | 16.99 | 94.49 |
| Comparative Example 4 | 16.32 | 14.32 | 87.75 |
| Comparative Example 5 | 17.86 | 15.64 | 87.57 |
| Comparative Example 6 | 18.05 | 17.11 | 94.79 |

The technical features of the above examples can be combined arbitrarily. In order to make the description concise, not all possible combinations of the technical features in the above examples are described. However, all the combinations of the technical features should be considered as falling within the scope recorded in this specification provided that they do not conflict with each other.

It should be noted that the present application is not limited to the above embodiments. The above embodiments are examples only, and any embodiment that has substantially the same constitutions and has the same effects as the technical ideas within the scope of the technical solutions of the present application is encompassed within the technical scope of the present application. The above embodiments merely represent some embodiments of the present application, giving details thereof, but should not be construed as limiting the scope of the patent thereby. In addition, without departing from the scope of the subject matter of the present application, various modifications that can be conceived of by those skilled in the art and are applied to the embodiments, and other embodiments constructed by combining some of the constituent elements of the embodiments are also encompassed within the scope of the present application. It should be noted that those of ordinary skill in the art may further make variations and improvements without departing from the concept of the present application, all of which fall within the scope of protection of the present application. Therefore, the scope of protection of the present application should be determined by the appended claims, and the specification and drawings can be used to explain the contents of the claims.

## Claims

1. A perovskite precursor solution, comprising a perovskite precursor material, a solvent and a surfactant, a structure of the surfactant comprising a hydrophilic group, a hydrophobic group and a first functional group, wherein the first functional group is a Lewis base group containing a lone pair, and the lone pair is present in at least one of an N atom and an S atom; and the first functional group, the hydrophilic group and the hydrophobic group are different from each other.

2. The perovskite precursor solution according to claim 1, wherein the first functional group comprises one or more of -NR¹¹-NR¹²R¹³ and -SH; wherein R¹¹ and R¹² are each independently H or hydrocarbyl; R¹³ is H, hydrocarbyl, or hydrocarbyl substituted with a monovalent hydrophilic group;
optionally, R¹¹ and R¹² are each independently H or alkyl, further optionally, R¹¹ and R¹² are each independently H or C₁₋₆ alkyl, still further optionally, R¹¹ and R¹² are each independently H or C₁₋₃ alkyl; still further optionally, R¹¹ and R¹² are each independently H or methyl; and still further optionally, R¹¹ and R¹² are both H;
optionally, R¹³ is H, alkyl or alkyl substituted with a monovalent hydrophilic group, further optionally, the alkyl or the alkyl substituted with the monovalent hydrophilic group is each independently C₁₋₂₀ alkyl, still further optionally C₁₋₁₈ alkyl, still further optionally C₁₋₁₅ alkyl, still further optionally C₁₋₁₂ alkyl, still further optionally C₁₋₁₀ alkyl, still further optionally C₁₋₈ alkyl, still further optionally C₁₋₆ alkyl, still further optionally C₁₋₄ alkyl, still further optionally C₁₋₃ alkyl, still further optionally methyl;
optionally, R¹³ is H or hydrocarbyl, further optionally H or alkyl, still further optionally H or C₁₋₂₀ alkyl, still further optionally H or C₁₋₁₈ alkyl, still further optionally H or C₁₋₁₅ alkyl, still further optionally H or C₁₋₁₂ alkyl, still further optionally H or C₁₋₁₀ alkyl, still further optionally H or C₁₋₈ alkyl, still further optionally H or C₁₋₆ alkyl, still further optionally H or C₁₋₄ alkyl, still further optionally H or C₁₋₃ alkyl, still further optionally H or methyl;
optionally, examples of the monovalent hydrophilic group may comprise, but are not limited to, one or more of: carboxyl, a sulfonic acid group, a sulfonate salt, a sulfuric acid group, a sulfate salt, a phosphoric acid group, a phosphate salt, a monohydrogen phosphate group, a monohydrogen phosphate salt, a primary amino group, a primary amino salt, a secondary amino group, a secondary amino salt, a quaternary ammonium salt, -CONH₂, and hydroxyl; and
optionally, R¹¹, R¹² and R¹³ are all H; still further optionally, the first functional group is selected from one or both of -NH-NH₂ and -SH.

3. The perovskite precursor solution according to claim 1 or 2, wherein the hydrophilic group comprises one or more of carboxyl, a sulfonic acid group, a sulfonate salt, a sulfuric acid group, a sulfate salt, a phosphoric acid group, a phosphate salt, a monohydrogen phosphate group, a monohydrogen phosphate salt, a primary amino group, a primary amino salt, a secondary amino group, a secondary amino salt, a divalent tertiary amino group, a quaternary ammonium salt, - CONH₂, hydroxyl, an ether group, an ester group, -CONH- and a divalent phosphate ester group;
optionally, the sulfonate salt, the sulfate salt, the phosphate salt and the monohydrogen phosphate salt are alkali metal salts of corresponding acids;
optionally, the primary amino salt and the secondary amino salt are salts formed by corresponding amine and acid; optionally, the acid is organic acid or inorganic acid; the organic acid comprises one or more of carboxylic acid, phosphonic acid, and sulfonic acid; the inorganic acid comprises one or more of hydrohalic acid, phosphoric acid, and sulfuric acid; and optionally, the inorganic acid comprises one or more of hydrochloric acid, hydroiodic acid, hydrobromic acid, hydrofluoric acid, and sulfuric acid; and
optionally, the hydrophilic group comprises at least one of quaternary ammonium ions and alkali metal ions.

4. The perovskite precursor solution according to any one of claim 3, wherein the hydrophilic group comprises one or more of *-COOH, *-S(=O)₂OH, *-S(=O)₂OM, *-O-S(=O)₂OH, *-O-S(=O)₂OM, *-O-(O=)P(OH)₂, 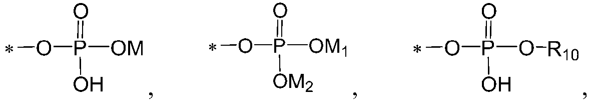 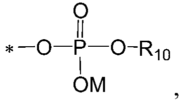 *-NH₂, *-NH₂·n₂A_{cd}, *-NHR₀, *-NHR₀·n₁A_{cd}, 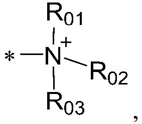 *-CONH₂, *-OH and a hydrophilic linker L₀; wherein the hydrophilic linker L₀ comprises one or more of *-CONH-*, *-NHCO-*, *-C(=O)O-*, *-O-C(=O)-*, *-O-(O=)P(OM₀₁)-O-*, *-NH-* *-O-*;
wherein any "*" indicates a connection site to a carbon atom;
any M is independently an alkali metal ion;
any M₁ and any M₂ are each independently an alkali metal ion;
any R₁₀ is independently hydrocarbyl;
any A_{cd} is independently an acid molecule; n₂ is 1 or 2; n₁ is 1;
any R₀ is independently hydrocarbyl or substituted hydrocarbyl; the substituted hydrocarbyl is substituted by one or more hydrophilic groups;
R₀₁ is alkyl; R₀₂ is hydrocarbyl; R₀₃ is alkyl; and
M₀₁ is absent or is H or an alkali metal ion.

5. The perovskite precursor solution according to claim 4, wherein the surfactant satisfies any one or more of the following features:
any M is independently lithium, sodium or potassium;
any M₁ and any M₂ are each independently lithium, sodium or potassium;
any R₁₀ is independently C₁₋₁₀ alkyl, optionally C₁₋₈alkyl, further optionally C₁₋₆ alkyl, still further optionally C₁₋₄ alkyl, still further optionally C₁₋₃ alkyl;
any A_{cd} is independently an organic acid or inorganic acid molecule; the organic acid comprises one or more of carboxylic acid, phosphoric acid, and sulfonic acid; the inorganic acid comprises one or more of hydrohalic acid and sulfuric acid; and optionally, the inorganic acid comprises one or more of hydrochloric acid, hydroiodic acid, hydrobromic acid, hydrofluoric acid, and sulfuric acid;
any R₀ is independently alkyl; optionally, any R₀ is independently C₁₋₃ alkyl; further optionally, R₀ is methyl;
R₀₁ is C₁₋₃ alkyl; optionally, R₀₁ is methyl;
R₀₂ is a C₁₋₈ alkyl or C₁₋₃ alkylene substituted with a benzene ring, and the benzene ring is phenyl or phenyl substituted with 1-4 C₁₋₃ alkyl; optionally, R₀₂ is methyl or benzyl;
R₀₃ is C₁₋₃ alkyl; optionally, R₀₃ is methyl;
M₀₁ is absent or is H, lithium, sodium or potassium; and
the monovalent hydrophilic group is connected to at least one side of the hydrophilic linker L₀.

6. The perovskite precursor solution according to any one of claims 1 to 5, wherein the hydrophobic group comprises a C₈₋₂₀carbon chain;
optionally, the C₈₋₂₀carbon chain is a linear or branched structure;
optionally, a main chain atom length of the C₈₋₂₀ carbon chain is in a range from 6 to 20; further optionally, the main chain atom length of the C₈₋₂₀carbon chain is in a range from 8 to 20; still further optionally, the main chain atom length of the C₈₋₂₀ carbon chain is in a range from 8 to 18; still further optionally, the C₈₋₂₀ carbon chain is a C₁₀₋₂₀ carbon chain with a main chain atom length ranging from 10 to 18;
optionally, the C₈₋₂₀ carbon chain is a saturated structure or an unsaturated structure;
optionally, the C₈₋₂₀ carbon chain is an aromatic chain or an aliphatic chain; and
optionally, a molecular structure of the surfactant comprises one or more C₈₋₂₀ carbon chains.

7. The perovskite precursor solution according to claim 6, wherein the hydrophobic group further comprises one or more of arylene Ar₀ and arylalkyl ArA;
optionally, the arylene Ar₀ is C₆₋₁₈ arylene, and further optionally, the arylene Ar₀ is phenylene or phenylene substituted with one or more C₁₋₃ alkyl; and
optionally, the aralkyl ArA is C₁₋₁₈ alkyl substituted with one or more aryl Ar₁, wherein any one of the aryl Ar₁ is independently phenyl or phenyl substituted with one or more C₁₋₄alkyl; further optionally, any one of the aryl Ar₁ is independently phenyl or phenyl substituted with one or more C₁₋₃ alkyl; still further optionally, any one of the aryl Ar₁ is independently phenyl or benzyl; and further optionally, the aralkyl ArA is benzyl.

8. The perovskite precursor solution according to any one of claims 1 to 7, wherein in one molecule of the surfactant, the number of the hydrophilic groups, the hydrophobic groups and the first functional groups is each independently one or more;
optionally, in one molecule of the surfactant, the number of the first functional groups is 1 or 2-5; and
optionally, in one molecule of the surfactant, the number of the hydrophilic groups is 1 or 2-5.

9. The perovskite precursor solution according to any one of claims 1 to 8, wherein a molar percentage of the first functional group relative to a divalent metal ion in the perovskite precursor material is in a range from 0.001 mol% to 5 mol%; and
optionally, the molar percentage of the first functional group relative to the divalent metal ion in the perovskite precursor material is in a range from 0.1 mol% to 2.5 mol%.

10. The perovskite precursor solution according to any one of claims 1 to 9, wherein the first functional group and the hydrophilic group are each independently connected to a carbon atom of the hydrophobic group.

11. The perovskite precursor solution according to any one of claims 1 to 10, wherein the surfactant comprises one or more of the following compounds: 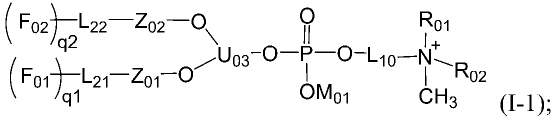
in formula (I-1), q1 and q2 are each independently 0 or a positive integer, q1+q2≥1, and L₂₁ and L₂₂ are each independently polyvalent hydrocarbyl having a main chain atom length of 8 to 20; Z₀₁ and Z₀₂ are each independently a covalent bond, carbonyl or -NHC(=O)-*, wherein * points to U₀₃; U₀₃ is trivalent hydrocarbyl; M₁₀is absent or is H or an alkali metal ion; L₁₀ is C₁₋₆ alkylene; R₀₁ is alkyl; R₀₂ is hydrocarbyl; in one molecule, at least one of F₀₁ and F₀₂ is the first functional group, and the other is independently H or the first functional group; 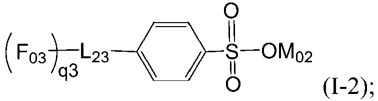
in formula (I-2), M₀₂ is an alkali metal ion; 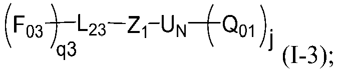
in formula (I-3), Z₁ is a covalent bond or a linker Z₁₀, wherein Z₁₀ is selected from any one of the following groups: -CO-NH, -NH-CO-, -C(=O)-O-, -O-C(=O)-, -NH-C(=O)-O-, -O-C(=O)-NH- and -O-; U_{N} is a j+1 valent group; j is a positive integer; any Q₀₁ is independently -OH or -COOM₀₃, M₀₃ is H or an alkali metal ion; 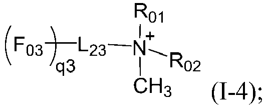
in formula (I-4), R₀₁ is alkyl; R₀₂ is hydrocarbyl;
in formula (I-2), formula (I-3), formula (I-4) and formula (I-5), F₀₃ is respectively and independently the first functional group; and
in formula (I-2), formula (I-3), formula (I-4) and formula (I-5), q3 is each independently an integer greater than 1, and L₂₃ is each independently polyvalent hydrocarbyl having a main chain atom length of 8 to 20.

12. The perovskite precursor solution according to claim 11, wherein the surfactant satisfies any one or more of the following features:
in formula (I-1), L₂₁ and L₂₂ are each independently a linear or branched type; optionally, L₂₁ and L₂₂ are each independently linear C₈₋₂₀ hydrocarbylene or branched C₈₋₂₀ polyvalent hydrocarbyl; further optionally, L₂₁ and L₂₂ are each independently linear C₈₋₂₀ hydrocarbylene; still further optionally, L₂₁ and L₂₂ are each independently linear C₈₋₂₀ alkylene or linear C₈₋₂₀ alkenylene; still further optionally, L₂₁ and L₂₂ are each independently linear C₁₀₋₁₈ alkylene or linear C₁₀₋₁₈alkenylene; still further optionally, L₂₁ and L₂₂ are each independently linear C₁₂₋₁₈ alkylene or linear C₁₂₋₁₈ alkenylene;
in formula (I-1), U₀₃is trivalent alkyl or 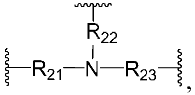 and R₂₁, R₂₂ and R₂₃ are each independently alkylene; optionally, U₀₃ is trivalent C₂₋₁₀ alkyl or trivalent C₃₋₁₀ tertiary amino, furtheroptionally, U₀₃ is CH₂₋CR₀₄(-)-CH₂-, >CH-L₀₄- or N(-CH₂CH₂-)₃, R₀₄ is H or C₁₋₄ alkyl (R₀₄ is further optionally H, methyl or ethyl, still further optionally H), L₀₄ is C₁₋₆ alkylene (L₀₄ is further optionally C₁₋₄ alkylene, still further optionally C ₁₋₄ alkylene, still further optionally methylene, 1,2-ethylidene, 1,3-propylidene or 1,4-butylidene); optionally, R₂₁, R₂₂ and R₂₃ are each independently C₁₋₄ alkylene, further optionally, R₂₁, R₂₂ and R₂₃ are each independently C₁₋₃ alkylene, still further optionally, R₂₁, R₂₂ and R₂₃ are each independently methylene or ethylidene, and still further optionally, R₂₁, R₂₂ and R₂₃ are all ethylidene;
in formula (I-1), M₀₁ is absent or is H, a lithium ion, a sodium ion or a potassium ion;
in formula (I-1), L₁₀ is C₁₋₄ alkylene, optionally ethylidene or propylidene, afurther optionally ethylidene;
in formula (I-1), R₀₁ is C₁₋₃ alkyl; optionally, R₀₁ is methyl;
in formula (I-1), R₀₂ is C₁₋₈ alkyl or C₁₋₃ alkylene substituted with a benzene ring, wherein the benzene ring is phenyl or phenyl substituted with 1-4 C₁₋₃ alkyl; optionally, R₀₂ is a methyl or benzyl;
in formula (I-2), M₀₂ is H, a lithium ion, a sodium ion or a potassium ion;
n formula (I-3), U_{N} is polyvalent hydrocarbyl containing 0, 1 or more hydrophilic linkers L₀₁, optionally, U_{N} is polyvalent saturated hydrocarbyl containing 0, 1 or more hydrophilic linkers L₀₁, any of the hydrophilic linkers L₀₁ is independently *-CONH-*, *-NHCO-*, *-C(=O)O-*, *-O-C(=O)-*, *-O-(O=)P(OM₀₁)-O-* or *-O-*, wherein * represents a connection site to a carbon atom, and M₀₁ is absent or is H, a lithium ion, a sodium ion or a potassium ion; alternatively, U_{N} is trivalent alkyl or tetravalent alkyl, optionally, the number of carbon atoms of U_{N} is in a range from 3 to 10, further optionally in a range from 3 to 6, further optionally 3, 4 or 5, still further optionally, U_{N} is -CH₂₋CR₀₃(-)-CH₂-, R₀₅ is H, methyl or ethyl, and still further optionally, R₀₅ is H; alternatively, U_{N} is trivalent tertiary amino, optionally trivalent C₃₋₁₀tertiary amino, and further optionally N(-CH₂CH₂-)₃;
in formula (I-3), the number of non-hydrogen atoms of U_{N} is in a range from 2 to 40, optionally in a range from 2 to 30, further optionally in a range from 2 to 25, still further optionally in a range from 2 to 20, still further optionally in a range from 2 to 18, still further optionally in a range from 2 to 15, still further optionally in a range from 2 to 12, still further optionally in a range from 2 to 10, still further optionally in a range from 2 to 8, still further optionally in a range from 2 to 6, still further optionally 2, 3, 4 or 5;
in formula (I-3), j is an integer selected from 1 to 5, optionally an integer selected from 1 to 4, further optionally 1, 2 or 3;
in formula (I-4), R₀₁ is C₁₋₃ alkyl; optionally, R₀₁ is methyl;
in formula (I-4), R₀₂ is C₁₋₈ alkyl or C₁₋₃ alkylene substituted with a benzene ring, wherein the benzene ring is phenyl or phenyl substituted with 1-4 C₁₋₃ alkyl; optionally, R₀₂ is a methyl or benzyl; and
in formula (I-2), formula (I-3) and formula (I-4), L₂₃ is respectively and independently a linear or branched type; optionally, L₂₃ is respectively and independently linear C₈₋₂₀ hydrocarbylene or branched C₈₋₂₀ polyvalent hydrocarbyl; further optionally, L₂₃ is respectively and independently linear C₈₋₂₀ alkylene or linear C₈₋₂₀ alkenylene; still further optionally, L₂₃ is respectively and independently linear C₁₀₋₁₈ alkylene or linear C₁₀₋₁₈ alkenylene; still further optionally, L₂₃ is respectively and independently linear C₁₂₋₁₈ alkylene or linear C₁₂₋₁₈ alkenylene.

13. The perovskite precursor solution according to claim 1, wherein the surfactant comprises one or more of the following compounds: 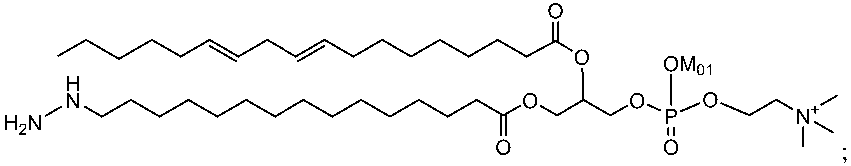 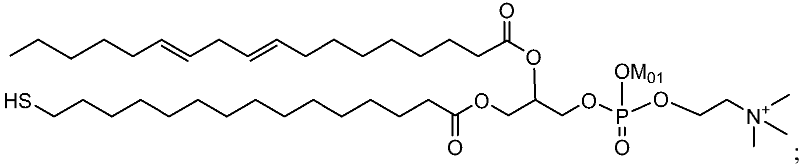 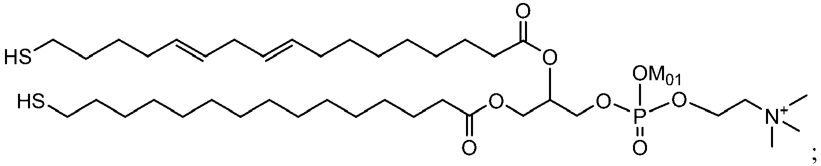 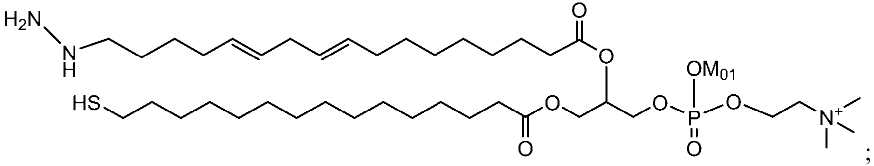 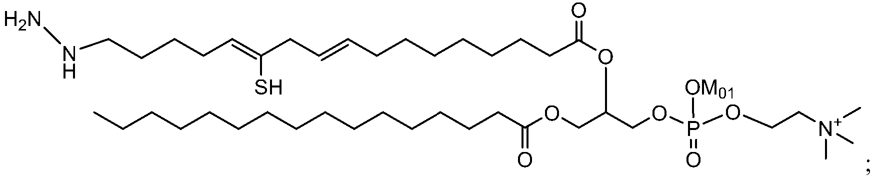 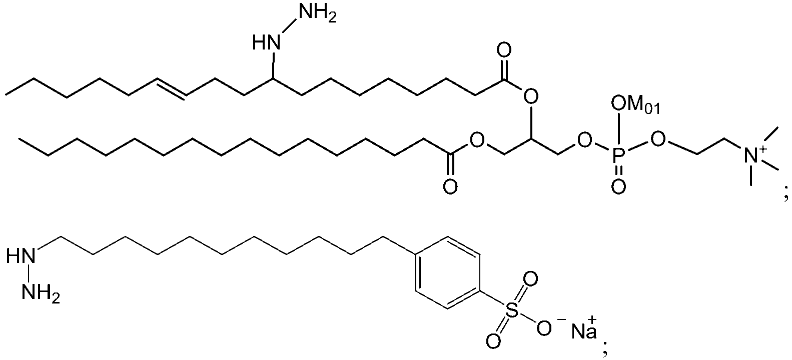 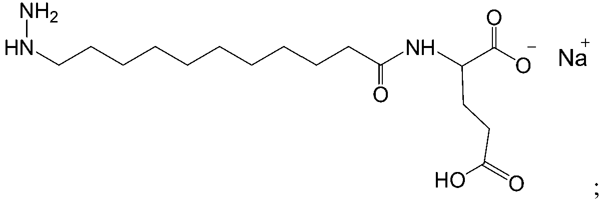 wherein M₀₁ is absent or is H or an alkali metal ion.

14. The perovskite precursor solution according to any one of claims 1 to 13, wherein a molar percentage of the surfactant relative to a divalent metal ion in the perovskite precursor material is in a range from 0.001 mol% to 5mol%;
optionally, the molar percentage of the surfactant relative to the divalent metal ion in the perovskite precursor material is in a range from 0.001% to 2.5%;
further optionally, the molar percentage of the surfactant relative to the divalent metal ion in the perovskite precursor material is in a range from 0.01% to 1%; and
still further optionally, the molar percentage of the surfactant relative to the divalent metal ion in the perovskite precursor material is in a range from 0.01% to 0.5%.

15. The perovskite precursor solution according to any one of claims 1 to 14, wherein the solvent comprises a first solvent and a second solvent, and a boiling point of the first solvent is lower than a boiling point of the second solvent.

16. The perovskite precursor solution according to claim 15, wherein the first solvent comprises one or more of N,N-dimethylformamide, 2-methoxyethanol and acetonitrile;
the second solvent comprises one or more of N-methylpyrrolidone, diphenyl sulfoxide, and dimethylpropyleneurea.

17. The perovskite precursor solution according to claim 15 or 16, wherein a volume ratio of the first solvent to the second solvent is in a range from 3 to 10;
Optionally, the volume ratio of the first solvent to the second solvent is in a range from 3 to 5.

18. A perovskite thin film, prepared by coating and annealing the perovskite precursor solution according to any one of claims 1 to 17, or at least containing a non-solvent component in the perovskite precursor solution according to any one of claims 1 to 16.

19. The perovskite thin film according to claim 18, wherein an area of the perovskite thin film is greater than or equal to 1 cm²;
optionally, the area of the perovskite thin film is greater than or equal to 4 cm².

20. A perovskite cell, comprising the perovskite thin film according to claim 18 or 19.

21. An electrical apparatus, comprising the perovskite cell according to claim 20.
